# EUROPEAN PATENT APPLICATION

(11) **EP 4 714 981 A1**
(43) Date of publication of application: **25.03.2026**
(21) Application number: 24810354.1
(22) Date of filing: 20.05.2024
(51) Int. Cl.: C07K 19/00, C12N 5/10, A61P 35/00, A61K 35/17, C07K 16/28

(54) **IMMUNE EFFECTOR CELL AND USE THEREOF**

(30) Priority: 19.05.2023 CN 202310574644
(71) Applicant: Carsgen Life Sciences Co., Ltd., Shanghai 200131 (CN)
(72) Inventor: JIANG, Hua, Shanghai 200131 (CN); LI, Zonghai, Shanghai 200131 (CN); DU, Guoxiu, Shanghai 200131 (CN); ZHANG, Honghong, Shanghai 200131 (CN)
(74) Representative: Michalski Hüttermann & Partner Patentanwälte mbB
(86) International application number: PCT/CN2024/094220
(87) International publication number: WO 2024/240112

(57) **Abstract**

Provided are an immune effector cell targeting CD19 and CD20, and use thereof. The immune effector cell comprises a scFv targeting CD19 and CD20, a hinge region, a transmembrane region, and an intracellular signaling domain. Further provided are a polynucleotide targeting CD19 and CD20, a corresponding expression vector, a CAR-T cell, and use thereof.

## Description

### Technical Field

The present invention relates to the field of biomedicine, and more specifically to immune effector cells targeting CD19 and CD20 and uses thereof.

### Background

Although anti-CD19 CAR-T therapy has shown a remarkable efficacy, there are still many problems for CD19 chimeric antigen receptor (CAR) T cell therapy, and some patients experience poor efficacy and prone to relapse. Therefore, there is an urgent need in the art to develop effective methods for treating tumors and preventing antigen escape.

### Summary of the Invention

The purpose of the present invention is to provide an immune effector cell capable of recognizing CD19 and CD20.

A first aspect of the present invention provides a multifunctional immune effector cell, wherein the immune effector cell comprises a chimeric receptor binding CD19 and CD20, or comprises a first chimeric receptor binding CD19 and a second chimeric receptor binding CD20, wherein the structure of the chimeric receptor binding CD19 and CD20 is represented by any one of the following formulas:
(1) L-V_{L1}V_{H2}-I-V_{L2}-V_{H1}-H-TM-C-CD3ζ
(2) L-V_{L2}-V_{H1}-V_{L1}V_{H2}-H-TM-C-CD3ζ
(3) L-V_{H1}-V_{L2}-I-V_{H2}-V_{L1}-H-TM-C-CD3ζ
(4) L-V_{H2}-V_{L2}-I-V_{L1}-V_{H1}-H-TM-C-CD3ζ

The structures of the first chimeric receptor binding CD19 and the second chimeric receptor binding CD20 are represented by any one of the following formulas:
(5) L-V_{L1}-V_{H1}-H-TM-C-CD3ζ and L-V_{H2}-V_{L2}-H-TM-C-CD3ζ
(6) L-V_{L1}-V_{H1}-H-TM-C-CD3ζ and L-V_{L2}-V_{H2}-H-TM-C-CD3ζ
(7) L-V_{H2}-V_{L2}-H-TM-C-CD3ζ and L-V_{H1}-V_{L1}-H-TM-C
(8) L-V_{H1}-V_{L1}-H-TM-C-CD3ζ and L-V_{H2}-V_{L2}-H-TM-C
(9) L-V_{H1}-V_{L1}-H-TM-C-CD3ζ and L-V_{H2}-V_{L2}-H-TM-C-CD3ζ

In the above formulas, each "-" independently represents a linker peptide or peptide bond; L is an optional signal peptide sequence; I is a flexible linker; H is an optional hinge region; TM is a transmembrane domain; C is a costimulatory signaling molecule; CD3ζ is an intracellular signaling sequence derived from CD3ζ; V_{H1} is the anti-CD19 antibody heavy chain variable region ; V_{L1} is the anti-CD19 antibody light chain variable region; V_{L2} is the anti-CD20 antibody light chain variable region; V_{H2} is the anti-CD20 antibody heavy chain variable region; and "-" is a linker peptide or peptide bond.

In one example, V_{H1} comprises the amino acid sequence shown in SEQ ID NO:21; V_{L1} comprises the amino acid sequence shown in SEQ ID NO:22; V_{H2} comprises the amino acid sequence shown in SEQ ID NO:23; V_{L2} comprises the amino acid sequence shown in SEQ ID NO:24.

In one example, the signal peptide is selected from: CD8α, GMCSF, GMCSFRα, IgGsL1, IgGsL2, IgGsL3, IgGsL4, IgGsH1, IgGsH2, IgGsH3, or IgGsH4 signal peptide; preferably, the signal peptide is selected from CD8α, or GMCSFRα signal peptide; and more preferably, the signal peptide comprises the amino acid sequence shown in SEQ ID NO:1 or 2.

In one example, the costimulatory signaling molecule is selected from: the intracellular domain of CD27, CD28, 4-1BB, OX40, CD30, CD40, PD-1, ICOS, or combinations thereof; preferably, the costimulatory signaling molecule is selected from: the intracellular domain of CD28, or 4-1BB; and more preferably, the costimulatory signaling molecule comprises the amino acid sequence shown in SEQ ID NO:10 or 11.

In one example, the transmembrane domain is selected from: the transmembrane domain of CD28, CD3, CD45, CD4, CD5, CD8, CD9, CD16, CD22, CD33, CD37, CD64, CD80, CD86, CD134, CD137, or CD154. Preferably, the transmembrane domain is selected from: the transmembrane domain of CD8, CD28; and more preferably, the transmembrane domain comprises the amino acid sequence shown in SEQ ID NO:7, 8, or 9.

In one example, the flexible peptide linker is selected from: newlinker or (G4S)n, wherein n is 1, 2, 3, 4, or 5; and preferably, the flexible peptide linker comprises the amino acid sequence shown in SEQ ID NO:14, 15, or 16.

In one example, the chimeric receptor comprises an amino acid sequence having at least about 80%, at least about 85%, at least about 90%, at least about 95%, at least about 96%, at least about 97%, at least about 98%, at least about 99%, or at least about 100% homology or identity to the amino acid sequence shown in SEQ ID NO:17, 29, 30, 31, 32, 33, 34, or 54, or a fragment thereof.

A second aspect of the present invention provides a polynucleotide, wherein the polynucleotide encodes the aforementioned chimeric receptor.

A third aspect of the present invention provides a chimeric receptor, wherein the chimeric receptor is a polypeptide chain, and its amino terminus is operably linked to a signal peptide that promotes secretion of antibody.

In one example, the signal peptide is selected from: natural IgG, IgM, IgD, IgA, or IgE signal peptide.

In one example, the chimeric receptor comprises an antigen-binding domain, which comprises the heavy chain variable region of the antibody and/or the light chain variable region of the antibody.

In one example, (i) the amino terminus of the chimeric receptor is the heavy chain variable region, and the amino terminus of the chimeric receptor is operably linked to the natural IgG, IgM, IgD, IgA, or IgE heavy chain signal peptide; or (ii) the amino terminus of the chimeric receptor is the light chain variable region, and the amino terminus of the chimeric receptor is operably linked to the natural IgG, IgM, IgD, IgA, or IgE light chain signal peptide.

In one example, the antigen-binding domain can bind one antigen, two or more epitopes of one antigen, or two or more different antigens.

In one example, the chimeric receptor comprises a transmembrane domain and an intracellular domain; preferably, the intracellular domain comprises an intracellular signaling domain; and more preferably, the intracellular domain further comprises a costimulatory signaling domain.

In one example, the chimeric receptor further comprises a spacer region located between the antigen-binding domain and the transmembrane domain.

In one example, the spacer region comprises a hinge region.

In one example, the hinge region comprises an IgG1, IgG2, IgG4 hinge region or a fragment thereof, a CD8 hinge region or a fragment thereof, or a CD28 hinge region or a fragment thereof.

In one example, the hinge region comprises the amino acid sequence encoded by the nucleic acid shown in SEQ ID NO:3, 4, 5, or 6.

In one example, the chimeric receptor comprises a chimeric antigen receptor (CAR) and a recombinant TCR receptor.

In one example, the antigen comprises a tumor antigen and/or a pathogen antigen; and preferably, the tumor antigen is selected from: CD19, CD20, BCMA, GPRC5D, GPC3, Claudin18.2, CD22, FAP, Mesothelin, NKG2D, NKG2A, CD94, CD38.

A fourth aspect of the present invention provides a polynucleotide, wherein the polynucleotide encodes the chimeric receptor described in the third aspect.

In one example, the chimeric receptor is a first chimeric receptor, and the polynucleotide further comprises a nucleotide sequence encoding a second chimeric receptor; and the first chimeric receptor comprises a first antigen-binding domain, and the second chimeric receptor comprises a second antigen-binding domain.

In one example, the first chimeric receptor and the second chimeric receptor are separated by one or more polycistronic components.

In one example, the signal peptide A linked to the first chimeric receptor and the signal peptide B linked to the second chimeric receptor are the same or different.

In one example, the polynucleotide, from N-terminus to C-terminus, comprises:
(i) a natural IgG, IgM, IgD, IgA, or IgE heavy chain signal peptide operably linked to the heavy chain variable region of the first antigen-binding domain; a natural IgG, IgM, IgD, IgA, or IgE heavy chain signal peptide operably linked to the heavy chain variable region of the second antigen-binding domain;
(ii) a natural IgG, IgM, IgD, IgA, or IgE heavy chain signal peptide operably linked to the heavy chain variable region of the first antigen-binding domain; a natural IgG, IgM, IgD, IgA, or IgE light chain signal peptide operably linked to the light chain variable region of the second antigen-binding domain;
(iii)a natural IgG, IgM, IgD, IgA, or IgE light chain signal peptide operably linked to the light chain variable region of the first antigen-binding domain; a natural IgG, IgM, IgD, IgA, or IgE heavy chain signal peptide operably linked to the heavy chain variable region of the second antigen-binding domain; or
(iv) a natural IgG, IgM, IgD, IgA, or IgE light chain signal peptide operably linked to the light chain variable region of the first antigen-binding domain; a natural IgG, IgM, IgD, IgA, or IgE light chain signal peptide operably linked to the light chain variable region of the second antigen-binding domain.

In one example, the polynucleotide, wherein the first and second antigen-binding domains can bind different epitopes of the same antigen, or the first and second antigen-binding domains bind different antigens.

In one example, the polynucleotide, wherein the nucleotide sequence of one or more domains selected from the spacer region, transmembrane domain, or intracellular domain of the second chimeric receptor is different from the nucleotide sequence encoding the corresponding domain of the first chimeric receptor; and the amino acid sequence transcribed from the nucleotide sequence encoding one or more domains selected from the spacer region, transmembrane domain, or intracellular domain of the second chimeric receptor is the same as the amino acid sequence of the corresponding domain of the first chimeric receptor.

In one example, the polynucleotide is codon-optimized for expression in human cells.

In one example, the polynucleotide, wherein the second antigen comprises a tumor antigen and/or a pathogen antigen; and preferably, the tumor antigen is selected from: CD19, CD20, BCMA, GPRC5D, GPC3, Claudin18.2, CD22, FAP, Mesothelin, NKG2D, NKG2A, CD94, or CD3.

A fifth aspect of the present invention provides an engineered cell comprising the nucleic acid molecule, vector, or chimeric receptor described above.

In one example, the engineered cell is an immune cell; and preferably, the immune cell is selected from: T cell, NK cell, cytotoxic T cell, NKT cell, dendritic cell, macrophage, CIK cell, stem cell-derived immune cell or combinations thereof.

In one example, the engineered cell is an autologous or allogeneic cell.

In one example, the engineered cell has low or no expression of endogenous TCR, B2M, HLA-II, and/or NKG2A.

A sixth aspect of the present invention provides a polynucleotide, wherein the polynucleotide encodes a chimeric receptor binding CD19 and CD20, or encoding a first chimeric receptor binding CD19 and a second chimeric receptor binding CD20; wherein the polynucleotide component encoding the chimeric receptor binding CD19 and CD20 is represented by any one of the following formulas:
(1) L-V_{L1}V_{H2}-I-V_{L2}-V_{H1}-H-TM-C-CD3ζ
(2) L-V_{L2}-V_{H1}-I-V_{L1}-V_{H2}-H-TM-C-CD3ζ
(3) L-V_{H1}-V_{L2}-I-V_{H2}-V_{L1}-H-TM-C-CD3ζ
(4) L-V_{H2}-V_{L2}-I-V_{L1}-V_{H1}-H-TM-C-CD3ζ

Wherein the polynucleotide component encoding the first chimeric receptor binding CD19 and the second chimeric receptor binding CD20 is represented by any one of the following formulas:
(5) L-V_{L1}-V_{H1}-H-TM-C-CD3ζ-2A-L-V_{H2}-V_{L2}-H-TM-C-CD3ζ
(6) L-V_{L1}-V_{H1}-H-TM-C-CD3ζ-2A-L-V_{L2}-V_{H2}-H-TM-C-CD3ζ
(7) L-V_{H2}-V_{L2}-H-TM-C-CD3ζ-2A-L-V_{H1}-V_{L1}-H-TM-C
(8) L-V_{H1}-V_{L1}-H-TM-C-CD3ζ-2A-L-V_{H2}-V_{L2}-H-TM-C
(9) L-V_{H1}-V_{L1}-H-TM-C-CD3ζ-2A-L-V_{H2}-V_{L2}-H-TM-C-CD3ζ

In the above formulas, each "-" independently represents a linker peptide or peptide bond component; L is an optional nucleic acid component encoding a signal peptide; I is a nucleic acid component encoding a flexible linker; H is an optional nucleic acid component encoding a hinge region; TM is a nucleic acid component encoding a transmembrane domain; C is a nucleic acid component encoding a costimulatory signaling molecule; CD3ζ is a cytoplasmic signaling sequence derived from CD3ζ; 2A is an optional 2A self-cleaving peptide; V_{H1} is the anti-CD19 antibody heavy chain variable region; V_{L1} is the anti-CD19 antibody light chain variable region; V_{L2} is the anti-CD20 antibody light chain variable region; V_{H2} is the anti-CD20 antibody heavy chain variable region; "-" is a linker peptide or peptide bond.

In one example, the polynucleotide is codon-optimized for expression in human cells.

In one example, the nucleotide sequence encoding the same component is codon-divergent.

In one example, V_{H1} comprises the amino acid sequence shown in SEQ ID NO:21; V_{L1} comprises the amino acid sequence shown in SEQ ID NO:22; V_{H2} comprises the amino acid sequence shown in SEQ ID NO:23; V_{L2} comprises the amino acid sequence shown in SEQ ID NO:24.

In one example, the signal peptide component is selected from: CD8α, GMCSF, GMCSFRα, IgGsL1, IgGsL2, IgGsL3, IgGsL4, IgGsH1, IgGsH2, IgGsH3, or IgGsH4 signal peptide; preferably, the signal peptide component is selected from CD8α, or GMCSF signal peptide; and more preferably, the signal peptide comprises the amino acid sequence shown in SEQ ID NO:1 or 2.

In one example, the costimulatory signaling molecule component is selected from: the intracellular domain of CD27, CD28, 4-1BB, OX40, CD30, CD40, PD-1, ICOS, or combinations thereof; preferably, the costimulatory signaling molecule component is selected from: the intracellular domain of CD28, or 4-1BB; and more preferably, the costimulatory signaling molecule comprises the amino acid sequence shown in SEQ ID NO:10 or 11.

In one example, the transmembrane domain component is selected from: the transmembrane domain of CD28, CD3, CD45, CD4, CD5, CD8, CD9, CD16, CD22, CD33, CD37, CD64, CD80, CD86, CD134, CD137, or CD154; preferably, the transmembrane domain component is selected from: the transmembrane domain of CD8, or CD28; and more preferably, the transmembrane domain comprises the amino acid sequence shown in SEQ ID NO:7, 8, or 9.

In one example, the flexible peptide linker is selected from: newlinker, or (G4S)n, wherein n is 1, 2, 3, 4, or 5; and preferably, the flexible peptide linker comprises the amino acid sequence shown in SEQ ID NO:14, 15, or 16.

In one example, the polynucleotide comprises the amino acid sequence having at least about 80%, 85%, 90%, 95%, 96%, 97%, 98%, 99%, or at least about 100% homology or identity to the amino acid sequence shown in SEQ ID NO:17, 29, 30, 31, 32, 33, 34, or 54, or fragment thereof.

A seventh aspect of the present invention provides a vector, wherein the vector comprises the polynucleotide described in the sixth aspect.

An eighth aspect of the present invention provides a chimeric receptor which comprises the amino acids encoded by the polynucleotide described in the sixth aspect, or the amino acids encoded by the vector described in the seventh aspect.

A ninth aspect of the present invention provides an engineered cell, wherein the engineered cell comprises the vector described in the seventh aspect, or the polynucleotide described in the sixth aspect, or expresses the chimeric receptor described in the eighth aspect.

In one example, the engineered cell is an immune cell; preferably, the immune cell is selected from: T cell, NK cell, cytotoxic T cell, NKT cell, dendritic cell, macrophage, CIK cell, stem cell-derived immune cell or combinations thereof; and more preferably, the engineered cell is an autologous or allogeneic cell.

In one example, the engineered cell has low or no expression of endogenous TCR, B2M, HLA-II, and/or NKG2A.

A tenth aspect of the present invention provides a method for preparing engineered cells, wherein the engineered cells express the aforementioned chimeric antigen receptor, comprising the following steps: (i) incubating an input composition comprising the cells to be transduced and a stimulator for the cells to be transduced for no more than 24 hours, (ii) then adding viral vector particles comprising the recombinant nucleic acid and incubating for no more than 24 hours to obtain the engineered cells.

In one example, the total incubation time for steps (i) and (ii) is no more than 48 hours, no more than 32 hours, no more than 28 hours, or no more than 24 hours.

An eleventh aspect of the present invention provides a medicament for preventing or treating tumors, comprising the aforementioned polynucleotide, vector, chimeric receptor, or engineered cell.

### Brief Description of Drawings

Figure 1 shows the structure of a dual-target chimeric antigen receptor binding CD19 and CD20: comprising signal peptide sequence (L), antigen recognition sequence recognizing CD19 and CD20, hinge region, transmembrane domian (TM), costimulatory factor signaling domain (Co-stimulator), and CD3ζ signaling transduction domain (CD3ζ). Expression vectors CAR6, 8, 9 as shown in Formula I; expression vector CAR10 as shown in Formula III; expression vector CAR7 as shown in Formula III; expression vector CAR11 as shown in Formula IX.
Figure 2 shows Daudi cells expressing CD19 and CD20; Nalm6 cells expressing CD19.
Figures 3A-3B show that CD19/CD20 dual-target CAR-T1, 2, 4, 5, 6, 7, 8, 9, 10 cells can all significantly kill tumor cells Daudi and Nalm6.
Figures 4A-4B show that after co-incubation with tumor cells Daudi or Nalm6, CD19/CD20 dual-target CAR-T1, 2, 4, 5, 6, 7, 8, 9, 10 cells can all secrete high levels of IFN-γ, IL-2, TNF-α.
Figure 5 shows the inhibition rate of dual-target CAR-T1, CAR-T11 on subcutaneous transplanted lymphoma (Daudi) in NPG mice.
Figure 6 shows that on Day 7 or Day 14 after infusion of dual-target CAR-T cells for the treatment of subcutaneously transplanted lymphoma (Daudi) in mice, surviving human T cells were detected in the peripheral blood of mice in both the CAR-T1 and CAR-T11 groups.
Figure 7 shows the treatment of orthotopic lymphoma in NPG mice with UTD, CD19-CAR-T, dual-target CAR-T4, 9, 10, or 12 cells.
Figure 8 shows IVIS imaging of tumor fluorescence intensity in NPG mice with orthotopic lymphoma treated with UTD, CD19-CAR-T, dual-target CAR-T4, 9, 10, or 12 cells.
Figure 9 shows the treatment of orthotopic lymphoma in NPG mice with UTD (TKO), CD19-CAR-T (DKO), CD20-CAR-T (DKO), CAR-T9 (DKO), CAR-T12 (DKO), or CAR-T9 (TKO) cells.
Figure 10 shows the survival status of mice on Day 131 after tumor cell infusion in NPG mice with orthotopic lymphoma treated with UTD (TKO), CD19-CAR-T (DKO), CD20-CAR-T (DKO), CAR-T9 (DKO), 20CAR-T12 (DKO), or CAR-T9 (TKO) cells.
Figure 11 shows IVIS imaging of tumor fluorescence intensity in NPG mice with orthotopic lymphoma treated with UTD (TKO), CD19-CAR-T (DKO), CD20-CAR-T (DKO), CAR-T9 (DKO), CAR-T12 (DKO), or CAR-T9 (TKO) cells.
Figure 12 shows that after co-incubation with tumor cells Daudi or Nalm6, both CAR9/NKG2A(1)-tko and CAR9/NKG2A(2)-tko cells can significantly kill tumor cells.
Figure 13 shows that after co-incubation with tumor cells Daudi and Nalm6, both CAR9/NKG2A(1)-tko and CAR9/NKG2A(2)-tko cells can secrete high levels of IL-2, TNF-α, and IFN-y cytokines.
Figure 14 shows that after co-incubation with human primary NK cells for 24 or 72 hours, both CAR9/NKG2A(1)-tko and CAR9/NKG2A(2)-tko cells can significantly kill NK cells.
Figure 15 shows that after co-incubation with NK cells, both CAR9/NKG2A(1)-tko or CAR9/NKG2A(2)-tko cells can secrete high levels of IL-2, TNF-α, and IFN-γ cytokines.

### Detailed Description

After extensive and in-depth research, the inventors unexpectedly discovered that immune effector cells expressing different forms of CD19/CD20 chimeric receptors exhibit varying tumor-killing abilities. The present invention was completed based on this discovery.

### Terminology

Unless specifically defined, all technical and scientific terms used herein have the same meaning as commonly understood by those skilled in the fields of gene therapy, biochemistry, genetics, and molecular biology. All methods and materials similar or equivalent to those described herein can be used in the practice or testing of the present invention, with suitable methods and materials described herein. All publications, patent applications, patents, and other references mentioned herein are incorporated by reference in their entirety. In case of conflict, this specification shall prevail. Furthermore, unless otherwise specified, the materials, methods, and examples of the present invention are illustrative and not intended to be limiting. Based on the content of the invention, those skilled in the art will understand that many variations or changes can be made to the disclosed specific embodiments and still achieve the same or similar results without departing from the spirit and scope of the invention. The scope of the invention is not limited to the specific embodiments described herein (which are intended only as illustrative examples of various aspects of the invention), and functionally equivalent methods and components are within the scope of the invention. The invention comprises modifications and variations of the subject matter for various uses and conditions.

Unless otherwise stated, the practice of the invention will employ conventional techniques in cell biology, cell culture, molecular biology, transgenic biology, microbiology, recombinant DNA, and immunology, all of which are within the technical scope of the field. These techniques are fully explained in the literature.

The term "about" refers to the usual error range of values easily known to those skilled in the art. As used herein, references to "about" a value or parameter comprises embodiments directed to the value or parameter itself. For example, a description of "about X" comprises a description of "X." As used herein, "about" may refer to an acceptable error range in the art. For example, it may refer to values or parameters within ±10% of the "about" value or parameter, for example, about 5 µM may comprise any number between 4.5 µM and 5.5 µM.

The term "CD19": CD19 (Gene ID: 930) encodes a member of the immunoglobulin gene superfamily. CD19 expression is restricted to B cell lymphocytes. This protein is a reliable marker of pre-B cells, and its expression decreases during terminal B cell differentiation in antibody-secreting plasma cells.

The term "CD20": CD20 (Gene ID: 931) encodes a B lymphocyte surface molecule that plays a role in the development and differentiation of B cells into plasma cells.

The term "FasL (Fas ligand)": (Gene ID: 356), is a member of the tumor necrosis factor superfamily, which activates the apoptotic signaling pathway by binding to Fas in target cells.

The term "NKG2A": NKG2A (Gene ID: 3821). NKG2A and CD94 form a dimer on NK cells and act as an inhibitory receptor for NK cells, and its ligand is HLA-E. NKG2A is generally expressed on NK cells and a small subset of CD8 T cells.

The term "CD38": CD38 (Gene ID: 952). It is expressed on the surface of many immune cells, comprising CD4 T, CD8 T, B lymphocytes, and NK cells. CD38 plays a role in cell adhesion, signal transduction, and calcium signaling.

The term "T cell receptor (TCR)" mediates the T cells recognition of specific major histocompatibility complex (MHC)-restricted peptide antigens, comprising classical TCR receptors and optimized TCR receptors. The classical TCR receptor consists of two peptide chains, α and β, each of which can be divided into a variable region (V region), a constant region (C region), a transmembrane region, and a cytoplasmic region, and its antigen specificity exists in the V region (Vα, Vβ), each of which has three hypervariable regions, CDR1, CDR2, and CDR3.

The term "recombinant T cell receptor (recombinant TCR)" comprises a chimeric receptor derived from one or more TCR subunits. For example, a recombinant TCR comprises at least a portion of the extracellular domain of a TCR subunit, transmembrane domain, and TCR intracellular domain, with the TCR subunit is effectively linked to the antigen-binding domain. For example, the TCR subunit in recombinant TCR may be derived from CD3ζ, CD3ε, CD3γ, CD38, TCRα, TCRβ, TCRγ, and/or TCRδ subunits. For example, recombinant TCR can be integrated into the TCR/CD3 complex expressed on T cells. For example, recombinant TCR comprises the constant region and intracellular domain of TCRα and TCRβ subunits, and the constant region of the subunit is effectively linked to the antigen-binding domain. For example, recombinant TCR comprises the constant region and intracellular domain of TCRγ and TCRδ subunits, and the constant region of the subunit is effectively linked to the antigen-binding domain. For example, recombinant TCR comprises the CD3ζ, CD3ε, CD3γ, or CD38 subunits, and the extracellular domain of the subunit is effectively linked to the antigen-binding domain.

The term "T cell antigen coupler (TAC)" comprises three functional domains: 1) an antigen-binding domain, comprising a single-chain antibody, a designed ankyrin repeat protein (DARPin), or other targeting moieties; 2) an extracellular domain, a single-chain antibody that binds to CD3, thereby bringing the TAC receptor close to the TCR receptor; 3) a transmembrane domain and the intracellular domain of the CD4 co-receptor, wherein the intracellular domain links to the protein kinase LCK, catalyzing phosphorylation of the immunoreceptor tyrosine-based activation motifs (ITAMs) of the TCR complex as the initial step of T cell activation.

The term "chimeric T cell receptor" comprises recombinant polypeptides derived from various polypeptides constituting the TCR, which are capable of binding to surface antigens on target cells and interacting with other polypeptides of the intact TCR complex, typically co-localized on the T cell surface. The chimeric T cell receptor consists of a TCR subunit and an antigen-binding domain composed of a human or humanized antibody domain, wherein the TCR subunit comprises at least a portion of the extracellular domain of a TCR subunit, transmembrane domain, and the stimulatory domain of the intracellular signaling domain of the TCR intracellular domain; the TCR subunit and antibody domain are effectively linked, wherein the extracellular, transmembrane, and intracellular signaling domains of the TCR subunit are derived from CD3ε or CD3γ, and the chimeric T cell receptor is integrated into the TCR expressed on T cells.

The term "chimeric antigen receptor (CAR)" comprises at least one extracellular antigen-binding domain, a transmembrane domain, and an intracellular signaling domain. The intracellular signaling domain comprises functional signaling domains of stimulatory molecules and/or co-stimulatory molecules. For example, the stimulatory molecule is derived from the ζ chain that binds to the T cell receptor complex (e.g., CD3). For example, the intracellular signaling domain further comprises a functional signaling domains of one or more co-stimulatory molecules, such as 4-1BB (CD137), CD27, and/or CD28. For example, polypeptide groups are linked to each other. For example, the intracellular signaling domain (or region) may be selected from the intracellular co-stimulatory domains of any one or more of the following polypeptides: CD27, CD28, TNFRSF9, TNFRSF4, TNFRSF8, TNFRSF14, TNFRSF18, CD40LG, ICOS, ITGB2, CD2, CD7, KLRC2, HAVCR1, LGALS9, CD83.

The term "primary signaling domain" or " primary signaling region " regulates the initial activation of the TCR complex in a stimulatory manner. In one aspect, the primary signaling is triggered by, for example, the binding of the TCR/CD3 complex to a peptide-loaded MHC molecule, thereby mediating T cell responses (including but not limited to proliferation, activation, differentiation, etc.). The primary signaling domain acting in a stimulatory manner may comprise immunoreceptor tyrosine-based activation motif, or a signaling motif of an ITAM. For example, fragments of primary signaling domains comprising ITAMs include but are not limited to intracellular signaling domains derived from CD3γ, CD3 δ , CD3ε, CD3ζ, CD5, CD79a, CD79b, CD278, and CD66d.

The term "signaling domain" or "signaling transduction domain" refers to the functional part of a protein that acts by transmitting information within the cell, used to regulate cellular activity via a defined signaling pathway by generating a second messenger or by acting as an effector in response to such messengers. The intracellular signaling domain may comprise the entire intracellular portion of the molecule, the entire native intracellular signaling domain, or functional fragments or derivatives thereof.

The term "co-stimulatory signaling domain" or "co-stimulatory molecule" usually refers to the intracellular domain of a co-stimulatory molecule that can combine with cell stimulatory signaling molecules, such as TCR/CD3, to result in T cell proliferation and/or upregulation or downregulation of key molecules. Co-stimulatory molecules are typically cognate binding partners on T cells that specifically bind co-stimulatory ligands, mediating co-stimulatory responses of T cells, including but not limited to proliferation. Co-stimulatory molecules are cell surface molecules other than antigen receptor or their ligands required for effective immune responses. Co-stimulatory molecules include but are not limited to MHC class I molecules, BTLA and Toll receptor ligands, as well as OX40, CD2, CD27, CD28, CDS, ICAM-1, LFA-1 (CD11a/CD18), and 4-1BB (CD137).

The term "CD3ζ (also known as CD3 Zeta)" comprises the protein provided by GenBank accession number BAG36664.1, or equivalent residues from non-human species such as mouse, rodent, monkey, ape, etc. In this application, "CD3ζ," "CD3z," and "CD3Z" are used interchangeably.

The term "newlinker" refers to a linker with the sequence GSTSGSGKPGSGEGSTKG (see Marc Whitlow et al., "An improved linker for single-chain Fv with reduced aggregation and enhanced proteolytic stability," Protein Engineering Vol.6 No.8 pp.989-995, 1993).

The term "cell" refers to cells of human or non-human, or animal origin.

The term "host" or "subject" refers to a recipient receiving a graft transplant, for example, an individual receiving exogenous cell implantation, such as a human. For example, "subject" may be a clinical patient, clinical trial volunteer, experimental animal, etc. The subject may be suspected of having a disease characterized by cell proliferation or may have, be diagnosed with, or be at risk for a disease characterized by cell proliferation. For example, the subject may have or be at risk for an immune disease such as autoimmune disease, tumor, or inflammatory disease.

The term "engineered" or "engineering" refers to the application of principles and methods of cell biology and molecular biology to alter the genetic material within cells or to obtain cell products at the whole-cell or organelle level as desired. For example, "engineered" refers to one or more alterations of nucleic acids (e.g., nucleic acids within the genome of an organism). "Engineered" may refer to gene alteration, addition, and/or deletion. "Engineered cells" may also refer to cells with added, deleted, and/or altered genes.

The term "immune cell" refers to cells involved in immune responses and producing immune effects, such as T cells, B cells, natural killer (NK) cells, natural killer T (NKT) cells, dendritic cells, CIK cells, macrophages, mast cells, etc. For example, the immune cell may be a T cell, NK cell, or NKT cell. For example, the T cell may be an autologous T cell, xenogeneic T cell, or allogeneic T cell. For example, the NK cell may be an autologous NK cell or allogeneic NK cell. For example, immune cells may be obtained by sorting peripheral blood mononuclear cells (PBMC) from a donor.

The term "T cell" may refer to natural T cells obtained from PBMC, bone marrow, lymph node tissue, cord blood, thymus tissue, or from infected sites, ascites, pleural effusion, spleen tissue, tumor tissue, or refer to cell populations with specific phenotypic characteristics obtained by sorting, or mixed cell populations with different phenotypic characteristics, such as "T cell" may comprise a cell population comprising at least one T cell subset: stem cell-like memory T cells (Tscm cells), central memory T cells (Tcm), effector T cells (Tef, Tef), regulatory T cells (Tregs), and/or effector memory T cells (Tem). In some cases, "T cell" may be a specific subtype of T cell, such as αβ T cells, γδ T cells. In some cases, T cells can be obtained from blood collected from individuals using any techniques known to those skilled in the art, such as FicollTM separation and/or apheresis. For example, T cells may be derived from induced pluripotent stem cells. For example, cells from circulating blood of an individual may be obtained by apheresis. Apheresis products typically contain lymphocytes, comprising T cells, monocytes, granulocytes, B cells, other nucleated white blood cells, red blood cells, and platelets. For example, cells collected by apheresis may be washed to remove plasma molecules and placed in a suitable buffer or medium for subsequent processing steps. The T cells may be derived from healthy donors or from cells derived from individuals diagnosed with cancer. T cells may be autologous or allogeneic. T cells may be primary T cells.

The term "cell composition" generally refers to a combination form comprising at least two types of cells, wherein the first type of cell can at least bind CD19 and CD20; the second type of cell binds NK cell markers. For example, each type of cell may be present in different containers and may be formulated into the desired preparation with suitable adjuvants simultaneously or separately as needed; each type of cell may be from different sources (e.g., prepared, produced, or sold by different manufacturers; e.g., naturally occurring T cells isolated from donors and T cells derived from stem cells, respectively); each type of cell may be prepared as an independent preparation (solid, liquid, gel, etc.); each type of cell may exist in mixed form. The cell composition may further comprise an effective amount of antibody, immune conjugate, chimeric receptor, nucleic acid, or host cell, and may also comprise a pharmaceutically acceptable carrier.

The term "MHC" refers to the major histocompatibility complex. In human cells, MHC is named as HLA antigen and plays an important role in transplant reactions, thereby mediating the rejection by T cells responding to the histocompatibility antigens on the surface of the transplanted tissue.

The term "human leukocyte antigen (HLA)" is the gene encoding the major histocompatibility complex in humans, closely related to the function of the human immune system. HLA comprises class I, class II, and class III gene segments. HLA class I is a heterodimer composed of a heavy chain (α chain) and a light chain β2-microglobulin (B2M). The term "B2M" refers to β-2 microglobulin, also known as B2M, which is the light chain of MHC class I molecules. HLA class II genes comprise the HLA-D family, mainly HLA-DP, HLA-DQ, and HLA-DR, mainly distributed on the surface of professional antigen-presenting cells such as B lymphocytes, macrophages, and dendritic cells.

The term "exogenous" refers to a nucleic acid molecule, polypeptide, cell, tissue, etc., that is not endogenously expressed in the organism, or whose expression level is insufficient to achieve the function of overexpression.

The term "endogenous" refers to a nucleic acid molecule or polypeptide, etc., originating from the organism itself.

The terms "activation" and "activating" or "stimulation" are used interchangeably to refer to the process by which a cell transitions from a resting state to an active state. This process may comprise a response to antigen and a response to phenotypic or genetic changes in migration, and/or functional activity. For example, "activation" may refer to the stepwise activation process of T cells. This activation process is jointly regulated by a primary stimulatory signal and a co-stimulatory signal. T cell activation is a dynamic process, with its duration and degree of activation influenced by external stimuli. "T cell activation" or "T cell stimulation" refers to the state of T cells stimulated to induce detectable cell proliferation, cytokine production, and/or detectable effector functions. The use of CD3/CD28 magnetic beads, *in vitro* antigen stimulation, or *in vivo* antigen stimulation will affect the degree and duration of T cell activation. For example, the engineered T cells are activated after co-incubation with tumor cells comprising specific target antigens or after viral infection.

The term "gene editing" or "genome editing" refers to gene engineering techniques that use site-specific nucleases to insert, knock out, modify, or replace DNA at specific locations in an organism's genome, thereby altering the DNA sequence. Gene editing can be used to achieve precise and efficient gene knockout or gene knock-in. Gene knockout techniques using nucleases comprise CRISPR/Cas technology, ZFN technology, TALEN technology, TALEN-CRISPR/Cas technology, base editor technology, Prime Editor technology, and Meganuclease technology. Guide RNA (gRNA) is a polynucleotide sequence sufficiently complementary to the target polynucleotide sequence to hybridize with the target sequence, and the gRNA is capable of directing the CRISPR complex to bind to the target sequence specifically. In this application, any gRNA sequence may be the targeted DNA sequence, or the complete Cas9 guide sequence formed by the ribonucleotide corresponding to the DNA along with crRNA and tracrRNA. gRNA is used to guide, bind, or recognize Cas enzymes. For example, engineered cells with endogenous TCR/B2M/FAS knockout or endogenous TCR/B2M knockout can be constructed using CRISPR technology. The gRNA sequences targeting TCR, B2M, FAS, and NKG2A are shown in SEQ ID NO: 35, 36, 37, and 38, respectively.

As used herein, "low expression" refers to the level of protein and/or RNA expressed by a target gene in engineered cells which is lower than its expression level prior to the cell engineering process. For example, low expression of B2M, TCR, NKG2A, or FAS refers to a reduction in the expression of B2M, TCR, NKG2A, or FAS in cells by at least 1%, at least 5%, at least 10%, at least 20%, at least 30%, at least 40%, at least 50%, at least 60%, at least 70%, at least 80%, at least 90%, at least 95%, at least 99%, or 100%. The expression or content of specific proteins in cells can be determined by any suitable method known in the art, such as ELISA, immunohistochemistry, immunoblotting, or flow cytometry using specific antibodies.

The term "transfection" refers to the introduction of exogenous nucleic acids into eukaryotic cells. Transfection can be achieved by various means known in the art, comprising calcium phosphate-DNA coprecipitation, DEAE-dextran-mediated transfection, polybrene-mediated transfection, electroporation, microinjection, liposome fusion, lipid transfection, protoplast fusion, retroviral infection, and biolistics.

The term "nucleic acid" or "nucleic acid molecule" refers to DNA or RNA in single-stranded or double-stranded form, and polymers thereof, comprising any nucleic acid molecule encoding a desired polypeptide or a fragment thereof.

The terms "nucleic acid molecule encoding," "encoding DNA sequence," and "encoding DNA" refer to the sequence or order of deoxyribonucleotides along a DNA strand. For example, a nucleic acid sequence encodes an amino acid sequence. When referring to nucleotide sequences, "sequence" may comprise DNA or RNA, and may be single-stranded or double-stranded.

The term "homology" or "identity" refers to the subunit sequence identity between two polymer molecules, e.g., between two nucleic acid molecules such as two DNA molecules or two RNA molecules, or between two polypeptide molecules. The term "substantial identity" or "substantial homology" refers to a polypeptide or nucleic acid molecule exhibiting at least about 50% homology or identity to a reference amino acid or nucleic acid sequence. Sequence identity can be measured using sequence analysis software (e.g., BLAST, BESTFIT, GAP, or PILEUP/PRETTYBOX programs). Such software matches identical or similar sequences by assigning degrees of homology to various substitutions, deletions, and/or other modifications.

The present invention provides amino acid or nucleic acid sequences with at least about 60%, 65%, 70%, 75%, 80%, 85%, 90%, 95%, 99%, or 100% homology or identity to the sequences shown in the sequence tables. The present invention provides amino acid or nucleic acid sequences based on the sequences shown in the sequence tables, which have been modified and/or substituted with one or several amino acids, and/or deleted and/or added with one or several amino acids, and have at least 60%, 65%, 70%, 75%, 80%, 85%, 90%, or 95% homology or identity to the sequences in the sequence tables.

The term "individual" refers to any animal, such as a mammal or marsupial. Individuals of the present invention include but are not limited to humans, non-human primates (e.g., rhesus monkeys or other types of macaques), mice, pigs, horses, donkeys, cattle, sheep, rats, and any kind of poultry.

The term "peripheral blood mononuclear cell (PBMC)" refers to cells in peripheral blood with a single nucleus, comprising lymphocytes, monocytes, etc. PBMC can be obtained by density-based cell separation methods, such as lysing red blood cells or not lysing red blood cells and subjecting peripheral blood, apheresis samples or leukapheresis samples by Percoll/Ficoll gradient centrifugation.

The term "effective amount" or "therapeutically effective amount" refers to a dose sufficient to prevent or treat a disease (tumor or cancer) in an individual. The effective dose for therapeutic or prophylactic use depends on the stage and severity of the disease being treated, the age, weight, and general health status of the subject, and the judgment of the prescribing physician. The magnitude of the dose also depends on the selected active substance, method of administration, timing and frequency of administration, the presence, nature, and extent of any adverse side effects accompanying the administration of a particular active substance, and the desired physiological effect. Based on the judgment from a prescribing physician or those skilled in the art, one or more rounds or multiple administrations of the engineered cells of this application may be required.

The term "expression vector" refers to a vector comprising recombinant polynucleotides, which comprises expression regulatory sequences effectively linked to the nucleotide sequence to be expressed. Expression vectors contain sufficient cis-acting elements for expression; and other elements for expression may be provided by the host cell or *in vitro* expression system. Expression vectors comprise plasmids, viruses (e.g., lentivirus, retrovirus, adenovirus, and adeno-associated virus).

The term "vector" refers to a composition comprising isolated nucleic acids and usable for delivering the isolated nucleic acids into the interior of cells. This includes but is not limited to linear polynucleotides, polynucleotides associated with ions or amphiphilic compounds, plasmids, and viruses. For example, it comprises autonomously replicating plasmids or viruses. It also comprises non-plasmid and non-viral compounds that facilitate the transfer of nucleic acid into cells, such as polylysine compounds, liposomes, etc.

The term "treatment" refers to interventions intended to alter the course of a disease, which may be preventive or may intervene during the clinical pathological process. Treatment effects include but are not limited to preventing the occurrence or recurrence of disease, alleviating symptoms, reducing any direct or indirect pathological consequences of the disease, preventing metastasis, slowing the rate of disease progression, improving or alleviating the condition, alleviating or improving prognosis, etc.

The term "prevention" refers to interventions attempted to be performed before the occurrence of a disease (such as tumor metastasis, recurrence).

The term "transplant immune rejection" refers to the immune response of a host to a foreign transplant, recognized as an "alien component." This response results in the transplant being attacked, destroyed, and cleared by the host's immune system after an allogeneic tissue, organ, or cell transplant is introduced.

The term "graft" refers to biological materials or preparations derived from individuals other than the host, intended for implantation into the host. The graft may be derived from any animal source, such as a mammalian source, preferably from humans. The graft may be derived from the host itself, such as host cells cultured or modified *in vitro* and then re-implanted into the host. The graft may also be derived from other allogeneic individuals, such as cells from other humans cultured or modified *in vitro* and implanted into the host.

The term "autologous" refers to originating from the same organism. For example, a sample (e.g., cells) may be taken from a subject, processed, and returned to the same subject at a later time. Allogeneic refers to the donor and recipient being different individuals.

The term "scFv" refers to a fusion protein comprising at least one antibody fragment that comprises a light chain variable region and at least one antibody fragment that comprises a heavy chain variable region, wherein the light chain variable regions and heavy chain variable regions are adjacent (e.g., connected by a synthetic linker, such as a short flexible polypeptide linker), and can be expressed as a single-chain polypeptide. The scFv retains the specificity of the intact antibody from which it is derived. Unless specified otherwise, as used herein, scFv may have the described VL and VH variable regions in any order (e.g., relative to the N-terminus and C-terminus of the polypeptide), and may comprise VL-linker-VH or VH-linker-VL. The antigen-binding function of an antibody may be performed by fragments of naturally occurring antibodies. These fragments are collectively referred to as "antigen-binding units." The term "antigen-binding unit" also comprises any molecular structure comprising polypeptide chains that has a specific shape suitable for recognizing an epitope, wherein one or more non-covalent binding interactions stabilize the complex between the molecular structure and the epitope.

The term "single domain antibody (sdAb)" may also be referred to as "VHH" or "VHH polypeptide," and comprises a variable VHH domain responsible for antigen recognition. The antigen binding of the VHH domain is mediated by three CDRs flanked by four relatively conserved framework regions (FRs). It refers to a class of antibodies lacking the light chain and comprising only the heavy chain variable region; due to its small molecular weight, it is also known as a nanobody. The VHH may be truncated at the N- terminus or C-terminus, so that it comprises only a part of FRI and/or FR4, or lacks one or two of those framework regions, as long as the VHH substantially maintains antigen binding and specificity.

The terms "recognize," "bind," and "target" are used interchangeably to refer to selective binding to a target antigen. For example, binding to a target cell refers to binding to a target antigen (e.g., target molecule) on the target cell.

The term "DDpp" refers to a target-binding D domain (DD) polypeptide based on a nonconventional antibody structural scaffold. The D domain polypeptide (DDpp) is characterized by a high target-binding affinity and a non-antibody structural scaffold. DDpp may be monovalent or multivalent. In some embodiments, DDpp is monospecific or multispecific. In other embodiments, it is monospecific and multivalent. In other embodiments, DDpp is multispecific and multivalent. For more information, see CN111727250A.

The term "variable region or variable domain" refers to the domain of the antibody heavy chain or light chain involved in antigen binding. The heavy chain variable domain (VH) and light chain variable domain (VL) of natural antibodies generally have similar structures, each comprising four conserved FRs and three CDRs. A single VH or VL domain may confer antigen-binding specificity. Furthermore, antibodies binding to specific antigens may be isolated by screening libraries of complementary VL or VH domains using the VH or VL domain from the antibody that binds the antigen, respectively.

The terms "hypervariable region," "complementarity-determining region," or "CDR" refer to regions in the variable domain of an antibody that are highly variable in sequence and/or form structurally defined loops ("hypervariable loops") and/or comprise residues that contact with the antigen ("antigen contact site"). Typically, antibodies comprise six CDRs: three in VH (HCDR1, HCDR2, HCDR3) and three in VL (LCDR1, LCDR2, LCDR3).

The term "Fc region" or "Fc" is used to define the C-terminal region of the immunoglobulin heavy chain comprising at least a portion of the constant region. This term comprises both native sequence Fc regions and variant Fc regions.

"Framework region (FR)" refers to variable domain residues other than those in the hypervariable region (CDR). The FR of the variable domain typically consists of four framework domains: FR1, FR2, FR3, and FR4. In VH (or VL), the CDR and FR sequences generally appear in the following order: FR1-HCDR1(LCDR1)-FR2-HCDR2(LCDR2)-FR3-HCDR3(LCDR3)-FR4.

Unless otherwise indicated, CDR residues and other residues in the variable domain (e.g., FR residues) are numbered according to Kabat et al.

The term "loop structure" generally refers to a curved, non-closed peptide segment, which may be formed by suitable linkage of anti-CD19 antigen-binding domain and anti-CD20 antigen-binding domain. For example, the anti-CD19 antibody heavy chain variable region (VH1) and anti-CD19 antibody light chain variable region (VL1) are linked to form antibody scFv1, and the anti-CD20 antibody heavy chain variable region (VH2) and anti-CD20 antibody light chain variable region (VL2) are respectively linked to both ends of antibody scFv1. For example, the anti-CD20 antibody heavy chain variable region (VH2) and anti-CD20 antibody light chain variable region (VL2) are linked to form antibody scFv2, and the anti-CD19 antibody heavy chain variable region (VH1) and anti-CD19 antibody light chain variable region (VL1) are respectively linked to both ends of antibody scFv2.

The terms "full antibody," "full-length antibody," and "complete antibody" are used interchangeably to refer to an antibody having a structure substantially similar to that of a native antibody or having a heavy chain of an Fc region as defined herein or comprising a complete full-length antibody with an antigen-binding region.

The term "fully human antibody" or "human antibody" refers to an antibody, the amino acid sequence of which corresponds to that of an antibody produced by a human or human cell, or is obtained from the amino acid sequence of an antibody from a non-human source using a human antibody library or other human antibody-encoding sequences.

The term "variant," refers to a different protein or polypeptide, compared with a protein or polypeptide having specific sequence characteristics ("control protein" or "reference polypeptide"), having one or more (e.g., about 1 to about 25, about 1 to about 20, about 1 to about 15, about 1 to about 10, or about 1 to about 5) amino acid substitutions, deletions, and/or additions. The amino acid sequence changes may be amino acid substitutions. The amino acid sequence changes may be conservative amino acid substitutions. Functional fragments or functional variants of the protein or polypeptide retain the basic structural and functional properties of the control protein or polypeptide.

The term "cell marker," also known as cell surface molecule or cell surface protein, preferably refers to molecules present on the membrane surface of immune cells. For example, "T cell and/or NK cell markers" refer to markers present on T cells or NK cells, or on both T cells and NK cells, including but not limited to: CD2, CD3, CD4, CD5, CD7, CD8, CD16a, CD16b, CD25, CD27, CD28, CD38, CD45, CD48, CD50, CD52, CD56, CD57, CD62L, CD69, CD94, CD100, CD102, CD122, CD127, CD132, CD160, CD161, CD178, CD218, CD226, CD244, CD159a (NKG2A), CD159c (NKG2C), NKG2E, CD314 (NKG2D), CD305, CD335 (NKP46), CD337, SLAMF7, TIGIT. For example, NK cell markers are selected from: NKG2 receptor family, killer immunoglobulin-like receptor (KIR) family, natural cytotoxicity receptor (NCR), and/or other antigens specifically expressed by NK cells. The NKG2 receptor family comprises NKG2A, NKG2D, NKG2C. The KIR family comprises KIR2DL1, KIR2DL2/3, KIR2DL4, KIR2DL5, KIR3DL1, KIR3DL2, KIR2DS1, KIR2DS2/S3, KIR2DS4, KIR2DS5, KIR3DS1. The NCR comprises NKP30, NKP44, NKP46, NKp80. Other antigens specifically expressed by NK cells comprise CD159a, CD159c, CD94, CD158, CD56, LIR/ILT2, CD244, CD226, CD2, CD16, CD161, TIGIT, CS1, IL-15R.

The term "NK inhibitory receptor (NKIR)" refers to a class of receptors on NK cells that can transduce killing-inhibitory signals and suppress the cytotoxic function of NK cells, comprising HLA-specific and non-HLA-specific inhibitory receptors. HLA-specific inhibitory receptors comprise CD94, NKG2A, and KIR. Non-HLA-specific NK inhibitory receptors comprise PD-1, Siglec7, LAIR1, and CD300A. NKIR includes, but is not limited to, immunoreceptor tyrosine-based inhibitory motif (ITIM). For example, NKIR comprises: NKG2/CD94 components, KIR family members, LIR family members, NKR-P1 family members, immune checkpoint receptors, immune checkpoint inhibitors, SIGLEC family members, Ly49 family members, or combinations thereof. For example, NKG2/CD94 components are selected from NKG2A, NKG2C, and CD94. For example, KIR family members are selected from KIR2DL1, KIR2DL2/3, KIR2DL5A, KIR2DL5B, KIR3DL1, KIR3DL2, and KIR3DL3. For example, LIR family members are selected from LIR1, LIR2, LIR3, LIR5, and LIR8. For example, NKR-P1 family members are selected from NKR-P1B and NKR-P1D. For example, immune checkpoint inhibitors comprise: (a) one or more antagonists of checkpoint molecules, comprising PD-1, PDL-1, TIM-3, TIGIT, LAG-3, CTLA-4, 2B4, 4-1BB, 4-1BBL, A2aR, BATE, BTLA, CD39, CD47, CD73, CD94, CD96, CD160, CD200, CD200R, CD274, CEACAM1, CSF-1R, Foxp1, GARP, HVEM, IDO, EDO, TDO, LAIR-1, MICA/B, NR4A2, MAFB, OCT-2, Rara (retinoic acid receptor α), TLR3, VISTA, NKG2A/HLA-E, or inhibitory KIR; (b) one or more of atezolizumab, avelumab, durvalumab, ipilimumab, IPH4102, IPH43, IPH33, lirentelimab, monalizumab, nivolumab, pembrolizumab and their derivatives or functional equivalents; or (c) at least one of atezolizumab, nivolumab, and pembrolizumab, or one or more of venetoclax, azacitidine, pomalidomide. For example, immune checkpoint receptors are selected from PD-1, TIGIT, CD96, TIM3, and LAG3. For example, SIGLEC family members are selected from SIGLEC7 and SIGLEC9. For example, Ly49 family members are selected from Ly49A, Ly49C, Ly49F, Ly49G1, and Ly49G4.

The term "pathological cell" generally refers to any type of cell present in a patient that is considered to cause deterioration of health, or refers to malignant or infected cells that need to be reduced or eliminated to achieve patient remission. The present invention relates to methods for novel adoptive immunotherapy strategies for treating diseases associated with the development of pathological cells, such as cancer (tumors), infections, and autoimmune diseases.

The term "tumor antigen" refers to antigens that newly appear or are overexpressed during the occurrence and development of hyperproliferative diseases.

The term "2A peptide" comprises sequences encoding self-cleaving peptides (e.g., 2A sequences) or protease recognition sites (e.g., furin protease). As used herein, "self-cleaving peptide" refers to an oligopeptide that allows multiple proteins to be encoded as a polyprotein, which dissociates into component proteins post-translation. This includes but is not limited to "F2A," "E2A," "P2A," and "T2A."

The term "signal peptide (L or SP)," also known as leader sequence, is a short peptide chain (about 5-30 amino acids in length) that directs newly synthesized proteins or polypeptides to the secretory pathway. The signal peptide is located at the N-terminus (amino terminus) of the protein. For example, SP is a CD8 signal peptide or GMCSFRα signal peptide. For example, SP is the natural signal peptide derived from a member of the wild-type immunoglobulin superfamily (IgSF). For example, SP is modified from the natural signal peptide of IgSF.

For example, the signal peptide is the natural IgG, IgM, IgD, IgA, or IgE heavy chain signal peptide. For example, the signal peptide is the natural κ or λ light chain signal peptide. For example, the SP linked to antibody VL is the natural κ light chain signal peptide, such as IgGsL1, IgGsL2, IgGsL3. For example, the signal peptide linked to antibody VL is the natural λ light chain signal peptide, such as IgGsL4.

The term "chimeric receptor" refers to a fusion molecule formed by linking DNA fragments or corresponding cDNAs of proteins from different sources using genetic recombination technology, which comprising an extracellular domain, a transmembrane domain, and an intracellular domain. Chimeric receptors include but are not limited to: chimeric antigen receptors (CAR), recombinant TCR receptors, synthetic polypeptide receptors (synNotch receptors). Recombinant TCR receptors comprise TAC and chimeric T cell receptors. In one example, the extracellular domain comprises an antigen-binding domain. In one example, the extracellular domain further comprises a hinge region.

The present invention discloses bispecific CARs targeting CD19 and CD20. When tumor cells undergo CD19 antigen escape or do not express CD19, the bispecific CAR clears tumor cells by recognizing CD20. Most leukemic tumor cells express CD20, comprising some CD19-negative patients after anti-CD19 CAR-T therapy. CD20, encoded by the MS4A gene, is a glycosylated protein and a B cell membrane marker, specifically expressed in more than 95% of normal and malignant B cells, which are at the pre-B cell stage and subsequent developmental stages; and the expression of CD20 did not stop until the cells differentiate into plasma cells. CD20 is used as another target for immunotherapy of B cell malignancies in the present invention. The present invention discloses a rationally optimized single-chain design and system, i.e., a bispecific CAR, which can be efficiently integrated into primary human T cells and can simultaneously target CD19 and CD20 upon T cell activation. The CAR-T cells of the present invention can recognize two antigens (CD19 and CD20). The present invention provides a highly effective potential method for preventing antigen escape.

A CAR that simultaneously targets CD19 and CD20 is used in the present invention. Compared with CARs targeting a single antigen, the affinity of the CAR is enhanced, T cell activity is improved, and these targets have additive or synergistic effects. CAR-T cells simultaneously targeting both CD19 and CD20 on the surface of tumor cells can reduce the possibility of antigen escape caused by downregulation or loss of a single surface antigen. The anti-CD19 antibody and anti-CD20 antibody are linked in a LOOP form. For example, the anti-CD19 antibody heavy chain variable region (VH1) and anti-CD19 antibody light chain variable region (VL1) are linked to form antibody scFv1, and the anti-CD20 antibody heavy chain variable region (VH2) and anti-CD20 antibody light chain variable region (VL2) are respectively linked to both ends of antibody scFv1. For example, the anti-CD20 antibody heavy chain variable region (VH2) and anti-CD20 antibody light chain variable region (VL2) are linked to form antibody scFv2, and the anti-CD19 antibody heavy chain variable region (VH1) and anti-CD19 antibody light chain variable region (VL1) are respectively linked to both ends of antibody scFv2.

The bispecific CAR with anti-CD19 antibody and anti-CD20 antibody sequences linked in LOOP form disclosed in the present invention, compared with bispecific CARs with anti-CD19 antibody and anti-CD20 antibody sequences linked in tandem form in the prior art, exhibits enhanced affinity, improved T cell activity, and stronger ability to kill or inhibit CD19 and/or CD20 positive cells (e.g., tumor cells) both *in vitro* and *in vivo.*

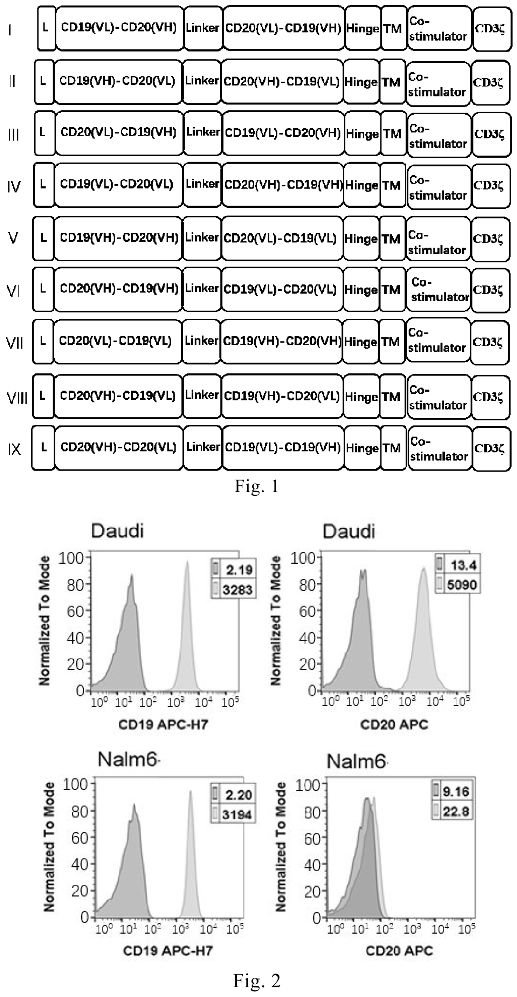

In one example, the chimeric receptor, from N-terminus to C-terminus, comprises an extracellular domain, transmembrane domain, and an intracellular signaling domain, wherein the extracellular domain comprises domains binding CD19 and/or CD20. The domain binding CD19 comprises an anti-CD19 antibody or a fragment thereof, or a synthetic binding domain. The domain binding CD20 comprises an anti-CD20 antibody or a fragment thereof, or a synthetic binding domain. The domain binding both CD19 and CD20 comprises an anti-CD19 and CD20 antibody or a fragment thereof, or a synthetic binding domain. The domains targeting CD19 and/or CD20 comprise antigen-binding units binding CD19 and/or CD20. For example, the chimeric receptor of the present invention comprises the chimeric receptors shown in Tables 1, 2, 3, or 4. For example, the chimeric receptor of the present invention comprises the chimeric receptor shown in Figure 1.

In one example, the anti-CD19 and/or CD20 antibody is selected from: full antibody, scFv, single domain antibody, Fab fragment, Fab' fragment, Fv fragment, F(ab')2 fragment, Fd fragment, sdAb, multifunctional antibody, DDPP antibody, scFv-Fc antibody, or IgG4 antibody. In one example, the anti-CD19 and/or CD20 antibody is scFv. In one example, the anti-CD19 and/or CD20 antibody is a single domain antibody. In one example, the anti-CD19 and/or CD20 antibody is a DDPP antibody.

In one example, the anti-CD19 antibody or a fragment thereof comprises a heavy chain variable region (VH1) and a light chain variable region (VL1). In one embodiment, VH1 comprises VH CDR1, VH CDR2, and VH CDR3, and VL1 comprises VL CDR1, VL CDR2, and VL CDR3, wherein VH CDR1, VH CDR2, and VH CDR3 are derived from VH with the amino acid sequence shown in SEQ ID NO:21, and VL CDR1, VL CDR2, and VL CDR3 are derived from VL with the amino acid sequence shown in SEQ ID NO:22.

In one example, the anti-CD20 antibody or a fragment thereof comprises a heavy chain variable region (VH2) and a light chain variable region (VL2). In one embodiment, VH2 comprises VH CDR1, VH CDR2, and VH CDR3, and VL2 comprises VL CDR1, VL CDR2, and VL CDR3, wherein VH CDR1, VH CDR2, and VH CDR3 are derived from VH2 with the amino acid sequence shown in SEQ ID NO:23, and VL CDR1, VL CDR2, and VL CDR3 are derived from VL2 with the amino acid sequence shown in SEQ ID NO:24.

In one example, the anti-CD19 antibody or a fragment thereof comprises VH1 and VL1. In one embodiment, VH1 and VL1 have the amino acid sequences shown in SEQ ID NOs:21 and 22, respectively, or have at least 90%, at least 95%, at least 98%, at least 99%, or 100% identity thereto.

In one example, the anti-CD20 antibody or a fragment thereof comprises VH2 and VL2. In one embodiment, VH2 and VL2 have the amino acid sequences shown in SEQ ID NOs:23 and 24, respectively, or have at least 90%, at least 95%, at least 98%, at least 99%, or 100% identity thereto.

It is well known in the art that there may be some amino acid substitutions in VH and VL region sequences while retaining the affinity for the target without changing the CDR sequences; and there also may be some amino acid substitutions in CDR sequences while retaining the affinity for the target without changing the amino acids contacting the target.

In one example, the anti-CD19 or CD20 antibody or a fragment thereof is scFv. The VH (heavy chain variable region) and VL (light chain variable region) of scFv may be linked by a linker peptide, and their positions may be interchangeable. In one example, the anti-CD19 or CD20 antibody comprises, from N-terminus to C-terminus, VH, linker peptide, and VL. In one example, the anti-CD19 or CD20 antibody comprises, from N-terminus to C-terminus, VL, linker peptide, and VH. Any linker peptide that connects VH and VL to form a functionally complete single-chain antibody can be used. In one example, the linker peptide is a GS linker, such as GGGGS, (GGGGS)3, or (GGGGS)4. In one example, the linker peptide is selected from SEQ ID NO:14, 15, or 16.

The chimeric receptor of the present invention, from N-terminus to C-terminus, comprises an extracellular domain, a transmembrane domain, and an intracellular signaling domain. When the intracellular signaling domain comprises one or more signaling domains or motifs, such as immunoreceptor tyrosine-based activation motif (ITAM), kinase domain, costimulatory domain, etc., it can directly promote cellular responses. The intracellular signaling domain may indirectly promote cellular responses by associating with one or more other proteins, which in turn directly promote cellular responses. The intracellular signaling domain or a functional fragment thereof may be derived from CD3ε, CD3δ, CD3ζ, CD25, CD27, CD28, CD40, CD47, CD79A, CD79B, CD134 (OX40), CD137 (4-1BB), CD150 (SLAMF1), CD278 (ICOS), CD357 (GITR), CARD11, DAP10, DAP12, FcRα, FcRβ, FcRγ, Fyn, Lck, LAT, LRP, NKG2D, NOTCH1, NOTCH2, NOTCH3, NOTCH4, ROR2, Ryk, Slp76, pTα, TCRα, TCRβ, TRIM, Zap70, PTCH2, or any combination thereof. The chimeric receptor provided by the present invention comprises an intracellular signaling domain. In one example, the intracellular signaling domain comprises an immunoreceptor tyrosine-based activation motif or a signaling motif of ITAM.

In one example, the intracellular signaling domain comprises the intracellular signaling domains selected from TCRα, TCRβ, TCRγ, TCRδ, CD3γ, CD3δ, CD3ε, CD3ζ, CD5, CD22, CD79a, CD79b, CD278, or CD66d, or combinations thereof. In one example, the intracellular signaling domain comprises the CD3ζ intracellular signaling domain (SEQ ID NO:12 or 13).

In one example, the intracellular signaling domain of the chimeric receptor provided by the present invention further comprises a costimulatory domain. In one example, the costimulatory domain is selected from the intracellular signaling domains of CD137, CD28, CD27, TNFRSF9, TNFRSF4, TNFRSF8, TNFRSF14, TNFRSF18, CD40LG, ICOS, ITGB2, CD2, CD7, KLRC2, HAVCR1, LGALS9, or CD83, or combinations thereof. In one example, the costimulatory domain is the intracellular signaling domain of CD137 (SEQ ID NO:11). In one example, the costimulatory domain is the intracellular signaling domain of CD28 (SEQ ID NO:10).

The chimeric receptor of the present invention comprises a transmembrane domain. In one example, the transmembrane domain is selected from the transmembrane domains of CD2, CD3ε, CD3δ, CD3ζ, CD8, CD25, CD27, CD28, CD40, CD79A, CD79B, CD80, CD86, CD95 (Fas), CD134 (OX40), CD137, CD150 (SLAMF1), CD152 (CTLA4), CD200R, CD223 (LAG3), CD270 (HVEM), CD272 (BTLA), CD273 (PD-L2), CD274 (PD-L1), CD278 (ICOS), CD279 (PD-1), CD300, CD357 (GITR), A2aR, DAP10, FcRα, FcRβ, FcRγ, Fyn, GAL9, KIR, Lck, LAT, LRP, NKG2D, NOTCH1, NOTCH2, NOTCH3, NOTCH4, PTCH2, ROR2, Ryk, Slp76, SIRPα, pTα, TCRα, TCRβ, TIM3, TRIM, LPA5, or Zap70. In one example, the transmembrane domain is the CD28 transmembrane domain (SEQ ID NO:8 or 9). In one example, the transmembrane domain is a variant of the CD28 transmembrane domain (SEQ ID NO:8 or 9) with no more than three amino acid mutations. In one example, the transmembrane domain is the CD8 transmembrane domain (SEQ ID NO:7). In one example, the transmembrane domain is a variant of the CD8 transmembrane domain (SEQ ID NO:7) with no more than three amino acid mutations.

In one example, in the chimeric receptor provided by the present invention, the extracellular domain and transmembrane domain are connected by a spacer region (also called hinge region or linker).

In one example, the hinge region is selected from: CD28 hinge region, CD8 hinge region, IgG spacer region or a fragment thereof, or combinations thereof. In one example, the hinge region may be an IgG1 spacer region or a fragment thereof, IgG2 spacer region or a fragment thereof, IgG3 spacer region or a fragment thereof, or IgG4 spacer region or a fragment thereof.

In one example, the hinge region is the CD8 hinge region (SEQ ID NO:3), IgG1 spacer region (SEQ ID NO:4), or IgG4 spacer region (SEQ ID NO:5 or 6).

In one example, the chimeric receptor comprises a signal peptide. In one example, the signal peptide is the CD8 signal peptide (SEQ ID NO:1) or GMCSFRα signal peptide (SEQ ID NO:2).

In one embodiment, the chimeric receptor provided by the present invention comprises: (1) a domain comprising an anti-CD19 antibody (VH/VL selected from SEQ ID NOs: 21, 22), and/or a domain comprising an anti-CD20 antibody (VH/VL selected from SEQ ID NOs: 23, 24); (2) a CD8 hinge region (SEQ ID NO: 3), an IgG1 spacer region (SEQ ID NO: 4), or an IgG4 spacer region fragment (SEQ ID NO: 5 or 6); (3) a CD28 transmembrane domain (SEQ ID NO: 8 or 9) or a CD8 transmembrane domain (SEQ ID NO: 7); (4) a CD28 intracellular signaling domain (SEQ ID NO: 10) or a CD137 intracellular signaling domain (SEQ ID NO: 11); optionally, also comprising (5) a CD3ζ intracellular signaling domain (SEQ ID NO: 12 or 13).

In one embodiment, the chimeric receptor provided by the present invention comprises an anti-CD19 antibody or a fragment thereof, and an anti-CD20 antibody or a fragment thereof; wherein the anti-CD19 antibody or a fragment thereof comprises a heavy chain variable region (VH1) and a light chain variable region (VL1), and the anti-CD20 antibody or a fragment thereof comprises a heavy chain variable region (VH2) and a light chain variable region (VL2). Different variable regions are connected by linker peptides. In one embodiment, the linker peptide comprises the amino acid sequences shown in SEQ ID NO: 14, 15, and/or 16.

The antigen-binding domain of the chimeric receptor may have different conformations, such as those shown in Figure 1. In one embodiment, the antigen-binding domain comprises, from the N-terminus to the C-terminus, VH1-VL1-VH2-VL2. In one embodiment, the antigen-binding domain comprises, from the N-terminus to the C-terminus, VL1-VH1-VL2-VH2. In one embodiment, the antigen-binding domain comprises, from the N-terminus to the C-terminus, VH1-VL1-VL2-VH2. In one embodiment, the antigen-binding domain comprises, from the N-terminus to the C-terminus, VL1-VH1-VH2-VL2. In one embodiment, the antigen-binding domain comprises, from the N-terminus to the C-terminus, VH1-VH2-VL2-VL1. In one embodiment, the antigen-binding domain comprises, from the N-terminus to the C-terminus, VL1-VH2-VL2-VH1. In one embodiment, the antigen-binding domain comprises, from the N-terminus to the C-terminus, VH1-VL2-VH2-VL1. In one embodiment, the antigen-binding domain comprises, from the N-terminus to the C-terminus, VL1-VL2-VH2-VH1. In one embodiment, the antigen-binding domain comprises, from the N-terminus to the C-terminus, VH2-VH1-VL1-VL2. In one embodiment, the antigen-binding domain comprises, from the N-terminus to the C-terminus, VL2-VH1-VL1-VH2. In one embodiment, the antigen-binding domain comprises, from the N-terminus to the C-terminus, VH2-VL1-VH1-VL2. In one embodiment, the antigen-binding domain comprises, from the N-terminus to the C-terminus, VL2-VL1-VH1-VH2.

The chimeric receptor provided by the present invention comprises an amino acid sequence with at least about 60%, 65%, 70%, 75%, 80%, 85%, 90%, 95%, 99%, or 100% homology or identity to the amino acid sequence as shown in SEQ ID NO: 17, 29, 30, 31, 32, 33, 34, or 55. The chimeric receptor provided by the present invention comprises an amino acid sequence with at least about 60%, 65%, 70%, 75%, 80%, 85%, 90%, 95%, 99%, or 100% homology or identity to the amino acid sequence as shown in SEQ ID NO: 39, 40, 41, or 42.

In one embodiment, the immune cell marker is NKG2A, CD38, CD7, TIGIT, FasL, or a combination thereof. In one embodiment, the chimeric receptor comprises an antibody or fragment thereof against NKG2A. In one embodiment, the antibody or fragment thereof is selected from: full antibody, scFv, single-domain antibody, Fab fragment, Fab' fragment, Fv fragment, F(ab')2 fragment, Fd fragment, sdAb, multifunctional antibody, DDPP antibody, scFv-Fc antibody, or IgG4 antibody. In one embodiment, the chimeric receptor binding the immune cell marker comprises an IgG4 hinge region. In one embodiment, the chimeric receptor binding the immune cell marker comprises an amino acid sequence with at least about 60%, 65%, 70%, 75%, 80%, 85%, 90%, 95%, 99%, or 100% homology or identity to the amino acid sequence as shown in SEQ ID NO: 39, 40, 41, or 42. For example, the chimeric receptor comprises amino acid sequences with at least about 60%, 65%, 70%, 75%, 80%, 85%, 90%, 95%, 99%, or 100% homology or identity to the amino acid sequences as shown in SEQ ID NO: 25 and 39, SEQ ID NO: 25 and 40, SEQ ID NO: 25 and 41, SEQ ID NO: 25 and 42, SEQ ID NO: 26 and 39, SEQ ID NO: 26 and 40, SEQ ID NO: 26 and 41, SEQ ID NO: 26 and 42, SEQ ID NO: 27 and 39, SEQ ID NO: 27 and 40, SEQ ID NO: 27 and 41, SEQ ID NO: 27 and 42, SEQ ID NO: 28 and 39, SEQ ID NO: 28 and 40, SEQ ID NO: 28 and 41, or SEQ ID NO: 28 and 42.

In one embodiment, the pathological cell is selected from malignant cells or infected cells. In one embodiment, the pathological cell is selected from CD19 and/or CD20 positive tumor cells or infected cells. In one embodiment, the pathological cell is selected from hematological tumor cells or pathological cells of autoimmune diseases. In one embodiment, the hematological tumor is selected from: leukemia, lymphoma, myeloma; and autoimmune diseases are selected from: multiple sclerosis, autoimmune liver disease, type 1 diabetes, systemic lupus erythematosus (SLE), rheumatoid arthritis (RA), ankylosing spondylitis (AS), Sjogren's syndrome (SS), polymyositis/dermatomyositis.

The sequences provided in this application are not limited to Bites binding CD19 and CD20; and the antibody sequences of the Bite binding CD19 and CD20 are connected in a LOOP form.

For example, the chimeric receptor further comprises a spacer region (also referred to as a hinge region or linker) located between the antigen-binding domain and the transmembrane binding domain. For example, the spacer region comprises a portion of an immunoglobulin. For example, the spacer region comprises the sequence of a hinge region, a CH2 region, and/or a CH3 region. For example, the spacer region comprises the entirety or a portion of the IgG4 hinge region and/or IgG2 hinge region, wherein the IgG4 hinge region is optionally a human IgG4 hinge region and the IgG2 hinge region is optionally a human IgG2 hinge region; the CH2 region comprises the entirety or a portion of IgG4 CH2 and/or IgG2 CH2, wherein IgG4 CH2 is optionally a human IgG4 CH2 and IgG2 CH2 is optionally a human IgG2 CH2; and/or the CH3 region comprises the entirety or a portion of IgG4 CH3 and/or IgG2 CH3, wherein IgG4 CH3 is optionally a human IgG4 CH3 and IgG2 CH3 is optionally a human IgG2 CH3. For example, the hinge region, CH2, and CH3 comprise the entirety or a portion of the hinge, CH2, and CH3 from human IgG4. For example, one or more of the hinge region, CH2, and CH3 are chimeric and comprise hinge, CH2, and CH3 from human IgG4 and human IgG2. For example, the spacer region comprises an IgG4/2 chimeric hinge region, or a modified IgG4 hinge region with at least one amino acid substitution compared to the human IgG4 hinge; an IgG2/4 chimeric CH2 region; and an IgG4 CH3 region.

The present invention provides chimeric receptors binding CD19 as shown in Table 1.

**Table 1. CD19-CAR Structure**

| Vector | Structure |
|---|---|
| CAR19.1 | L-CD19V_{H}-CD19V_{L}-CD8(H)-CD8(TM)-CD137(C) |
| CAR19.2 | L-CD19V_{H}-CD19V_{L}-CD8(H)-CD28(TM)-CD28(C)-CD3ζ(C) |
| CAR19.3 | L-CD19V_{L}-CD19V_{H}-IgG4(H)-CD28(TM)-CD 137(C)-CD3ζ(C) |
| CAR-19 | L-CD19V_{L}-CD19V_{H}-CD8(H)-CD8(TM)-CD137(C) |

The present invention provides chimeric receptors binding CD20 as shown in Table 2.

**Table 2. CD20-CAR Structure**

| Vector | Structure |
|---|---|
| CAR20.4 | L-CD20V_{H}-CD20V_{L}- CD8(H)-CD28(TM)-CD28(C)-CD3ζ |
| CAR20.5 | L-CD20V_{H}-CD20V_{L}- CD8(H)-CD8(TM)-CD137(C) |
| CAR20.6 | L-CD20V_{H}-CD20V_{L}-CD8(H)-CD28(TM)-CD28(C)-CD137(C) |
| CAR20.7 | L-CD20V_{H}-CD20V_{L}- IgG4 (H)-CD28(TM)-CD137(C)- CD3C |
| CAR20.8 | L-CD20V_{L}-CD20V_{H}- IgG4 (H)-CD28(TM)-CD137(C)- CD3C |
| CD20-BBZ | L-CD20V_{L}-CD20V_{H}-CD8(H)-CD8(TM)-CD137(C) |

The present invention provides chimeric receptors binding both CD19 and CD20 as shown in Table 3.

**Table 3. CD19-CD20-CAR Structure**

| Vector | Structure |
|---|---|
| CAR6 | L-CD19V_{L}-CD20V_{H}-I₁-CD20V_{L}-CD19V_{H}-CD8(H)-CD8(TM)-CD137(C)- CD3ζ |
| CAR7 | L-CD20V_{L}-CD19V_{H}-I₁-CD19V_{L}-CD20V_{H}-CD8(H)-CD8(TM)-CD137(C)- CD3ζ |
| CAR8 | L-CD19V_{L}-CD20V_{H}-I₂-CD20V_{L}-CD19V_{H}-CD8(H)-CD8(TM)-CD137(C)- CD3ζ |
| CAR9 | L-CD19V_{L}-CD20V_{H}-I₂-CD20V_{L}-CD19V_{H}-IgG4(H)-CD28(TM)-CD137(C)- CD3ζ |
| CAR10 | L-CD19V_{H}-CD20V_{L}-I₂-CD20V_{H}-CD19V_{L}-IgG4(H)-CD28(TM)-CD137(C)- CD3ζ |
| CAR11 | L-CD20V_{H}-CD20V_{L}-I₃-CD19V_{L}-CD19V_{H}-D8(H)-CD8(TM)-CD137(C)- CD3ζ |
| CAR12 | L-CD20V_{L}-CD20V_{H}-I₃-CD19V_{H}-CD19V_{L}-IgG4(H)-CD28(TM)-CD137(C)- CD3ζ |

The present invention provides chimeric receptors binding immune cell markers as shown in Table 4.

**Table 4. CAR Structures Targeting Immune Cell Markers**

| CAR Vector | Structure |
|---|---|
| NKG2A-s28z | L-NKG2A(scFv)-IgG4(H)-CD28(TM)-CD28(C)-CD3ζ |
| NKG2A-28z | L-NKG2A(scFv)-CD8(H)-CD28(TM)-CD28(C)-CD3C |
| CD38-s28z | L-CD38(scFv)-IgG4(H)-CD28(TM)-CD28(C)-CD3ζ |
| CD38-28z | L-CD38(scFv)-CD8(H)-CD28(TM)-CD28(C)-CD3ζ |
| FasL-s28z | L-FasL(scFv)-IgG4(H)-CD28(TM)-CD28(C)-CD3ζ |
| FasL-28z | L- FasL(scFv)-CD8(H)-CD28(TM)-CD28(C)-CD3C |

In the above Tables 1, 2, 3, and 4: L: CD8 signal peptide (SEQ ID NO: 1), or GMCSFRα signal peptide (SEQ ID NO: 2); CD8(H): CD8α hinge (SEQ ID NO: 3); IgG4(H): IgG4 short hinge (SEQ ID NO: 6); I₁ (SEQ ID NO: 14), I₂ (SEQ ID NO: 15), I₃ (SEQ ID NO: 16); CD8TM (SEQ ID NO: 7), CD28TM (SEQ ID NO: 8 or 9); CD137(C): CD137 co-stimulatory signaling domain (SEQ ID NO: 11); CD28(C): CD28 co-stimulatory signaling domain (SEQ ID NO: 10); CD3ζ: CD3ζ signaling domain (SEQ ID NO: 12 or 13); CD19V_{H}: anti-CD19 antibody VH (SEQ ID NO: 21); CD19V_{L}: anti-CD19 antibody VL (SEQ ID NO: 22); CD20V_{H}: anti-CD20 antibody VH (SEQ ID NO: 23); CD20V_{L}: anti-CD20 antibody VL (SEQ ID NO: 24); NKG2A(scFv): anti-NKG2A antibody scFv (SEQ ID NO: 25); CD38(scFv): anti-CD38 antibody scFv (SEQ ID NO: 28); TIGIT(scFv): anti-TIGIT antibody scFv (SEQ ID NO: 27); FasL(scFv): anti-FasL antibody scFv (SEQ ID NO: 26). Corresponding lentiviruses expressing the above vectors are prepared using conventional molecular biology techniques.

The CAR-T1 cells disclosed in the embodiments of the present invention comprise CAR19.1 and CAR20.4 from Tables 1 and 2; CAR-T2 cells comprise CAR19.2 and CAR20.5 from Tables 1 and 2; CAR-T3 cells comprise CAR19.2 and CAR20.6 from Tables 1 and 2; CAR-T4 cells comprise CAR19.3 and CAR20.7 from Tables 1 and 2; CAR-T5 cells comprise CAR19.3 and CAR20.8 from Tables 1 and 2; CAR-T6 cells comprise CAR6 from Table 3; CAR-T7 cells comprise CAR7 from Table 3; CAR-T8 cells comprise CAR8 from Table 3; CAR-T9 cells comprise CAR9 from Table 3; CAR-T10 cells comprise CAR10 from Table 3; CAR-T11 cells comprise CAR11 from Table 3. The control group CAR-12 comprises CAR12 from Table 3. CAR9/NKG2A(1) T cells comprise CAR9 and NKG2A-28z from Tables 3 and 4. CAR9/NKG2A(2) T cells comprise CAR9 and NKG2A-s28z from Tables 3 and 4.

### Vectors

Genetic modification of immune cells (e.g., T cells or NKT cells) in the composition can be accomplished by transducing a substantially homogeneous cell population with recombinant nucleic acid molecules. In one embodiment, retroviral vectors (γ-retrovirus or lentivirus) are used to introduce nucleic acid molecules into cells. For example, polynucleotides encoding exogenous receptors (e.g., CARs) can be cloned into retroviral vectors. Non-viral vectors may also be used. Transduction can be performed using any suitable viral vector or non-viral delivery system. CARs can be constructed with auxiliary molecules (e.g., cytokines) in a single polycistronic expression cassette, multiple expression cassettes in a single vector, or multiple vectors. Examples of elements for generating polycistronic expression cassettes include, but are not limited to, various viral and non-viral internal ribosome entry sites (IRES, e.g., FGF-1 IRES, FGF-2 IRES, VEGF IRES, IGF-II IRES, NF-κB IRES, RUNX1 IRES, p53 IRES, hepatitis A IRES, hepatitis C IRES, pestivirus IRES, baculovirus IRES, picornavirus IRES, poliovirus IRES, and encephalomyocarditis virus IRES) and cleavable linkers (e.g., 2A peptides, such as P2A, T2A, E2A, and F2A peptides).

Other viral vectors that can be used comprise, for example, adenovirus, lentivirus, adeno-associated virus vectors, vaccinia virus, bovine papillomavirus, or herpesvirus, such as Epstein-Barr virus.

Non-viral methods can also be used for genetic modification of immune cells. For example, nucleic acid molecules can be introduced into immune cells by lipid transfection, asialoorosomucoid-polylysine conjugation, or microinjection under surgical conditions. Other non-viral gene transfer methods comprise *in vitro* transfection using liposomes, calcium phosphate, DEAE-dextran, electroporation, and protoplast fusion. Nucleic acid molecules may also be first transferred into cell types that can be cultured *ex vivo* (e.g., autologous or allogeneic primary cells or their progeny), and then the cells modified by the nucleic acid molecules (or their progeny) are injected into the target tissue of the subject or administered systemically.

The present invention provides nucleic acid molecules encoding chimeric receptors as shown in Tables 1, 2, 3, or 4.

The present invention provides nucleic acid molecules encoding any CD19-CAR molecule as shown in Table 1 and any CD20-CAR molecule as shown in Table 2. In one embodiment, the polypeptide chain encoded by the nucleic acid molecule comprises, in order from the amino terminus (N-terminus) to the carboxyl terminus (C-terminus), CD19-CAR and CD20-CAR; or comprises CD20-CAR and CD19-CAR in sequence. In one embodiment, the CAR binding CD19 and the CAR binding CD20 are connected by a hydrolyzable linker peptide. In one embodiment, the hydrolyzable linker peptide is P2A (SEQ ID NO: 18), E2A (SEQ ID NO: 20), or F2A (SEQ ID NO: 19).

The present invention provides nucleic acid molecules encoding chimeric receptors binding CD19 and CD20 as shown in Table 3, and chimeric receptors binding immune cell markers as shown in Table 4. In one embodiment, the polypeptide chain encoded by the nucleic acid molecule comprises, in order from the amino terminus (N-terminus) to the carboxyl terminus (C-terminus), a CAR binding CD19 and CD20, and a chimeric receptor binding an immune cell marker; or comprises a chimeric receptor binding an immune cell marker and a CAR binding CD19 and CD20 in sequence. In one embodiment, the polypeptide chain encoded by the nucleic acid molecule comprises, in order from the amino terminus (N-terminus) to the carboxyl terminus (C-terminus), a CAR binding CD19 and CD20, and a chimeric receptor binding NKG2A; or comprises a chimeric receptor binding NKG2A and a CAR binding CD19 and CD20 in sequence. In one embodiment, the polypeptide chain encoded by the nucleic acid molecule comprises, in order from the amino terminus (N-terminus) to the carboxyl terminus (C-terminus), a CAR binding CD19 and CD20, and a chimeric receptor binding TIGIT; or comprises a chimeric receptor binding TIGIT and a CAR binding CD19 and CD20 in sequence. In one embodiment, the polypeptide chain encoded by the nucleic acid molecule comprises, in order from the amino terminus (N-terminus) to the carboxyl terminus (C-terminus), a CAR binding CD19 and CD20, and a chimeric receptor binding CD38; or comprises a chimeric receptor binding CD38 and a CAR binding CD19 and CD20 in sequence. In one embodiment, the polypeptide chain encoded by the nucleic acid molecule comprises, in order from the amino terminus (N-terminus) to the carboxyl terminus (C-terminus), a CAR binding CD19 and CD20, and a chimeric receptor binding FasL; or comprises a chimeric receptor binding FasL and a CAR binding CD19 and CD20 in sequence.

The present invention provides nucleic acid molecules encoding a CAR binding CD19 and CD20, and a CAR binding immune cell markers. For example, the nucleic acid molecule encodes any CAR molecule binding CD19 and CD20 as shown in Table 3, and any CAR molecule binding immune cell markers as shown in Table 4. In one embodiment, the CAR binding CD19 and CD20 and the CAR binding immune cell markers are connected by a hydrolyzable linker peptide. In one embodiment, the immune cell markers comprise: NKG2A, CD38, TIGIT, and/or FasL.

The present invention provides nucleic acid molecule 3 encoding a CAR binding CD19 and CD20, and nucleic acid molecule 4 encoding a CAR binding immune cell marker. In one embodiment, nucleic acid molecules 3 and 4 are constructed on one vector. In one embodiment, the hydrolyzable linker peptide is P2A (SEQ ID NO: 18), E2A (SEQ ID NO: 20), or F2A (SEQ ID NO: 19). In one embodiment, the CAR binding CD19 and CD20 and the CAR binding immune cell marker are connected by a hydrolyzable linker peptide. In one embodiment, nucleic acid molecules 3 and 4 are constructed on different vectors. In one embodiment, the immune cell markers comprise: NKG2A, CD38, TIGIT, and/or FasL.

### Engineered Cells

The present invention provides an engineered cell comprising the chimeric receptor disclosed in the present invention.

In one embodiment, the engineered cell comprises a chimeric receptor binding CD19 (as shown in Table 1), and further comprises a chimeric receptor binding CD20 (as shown in Table 2).

In one embodiment, the engineered cell comprises a chimeric receptor binding CD19 (as shown in Table 1), a chimeric receptor binding CD20 (as shown in Table 2), and a chimeric receptor binding immune cell markers (as shown in Table 4).

In one embodiment, the engineered cell comprises a chimeric receptor binding CD19 and CD20 (as shown in Table 3).

In one embodiment, the engineered cell comprises a chimeric receptor binding CD19 and CD20 (as shown in Table 3), and further comprises a chimeric receptor binding immune cell markers (as shown in Table 4).

In one embodiment, the pathological cell is selected from malignant cells or infected cells. In one embodiment, the pathological cell is selected from hematological tumor cells or pathological cells of autoimmune diseases. In one embodiment, the hematological tumor cell is a CD19 positive and/or CD20 positive tumor cell.

In one embodiment, the hematological tumor is selected from: leukemia, lymphoma, myeloma; autoimmune diseases are selected from: multiple sclerosis, autoimmune liver disease, type 1 diabetes, systemic lupus erythematosus (SLE), rheumatoid arthritis (RA), ankylosing spondylitis (AS), Sjogren's syndrome (SS), polymyositis/dermatomyositis.

In one embodiment, the present invention provides an engineered cell comprising a polynucleotide encoding as disclosed in the present invention. In one embodiment, the present invention provides an engineered cell comprising a vector encoding as disclosed in the present invention. In one embodiment, the present invention provides an engineered cell comprising a virus encoding as disclosed in the present invention.

In one embodiment, the engineered cell is an immune cell, neuron, epithelial cell, endothelial cell, or stem cell. Stem cells comprise human pluripotent stem cells (comprising human induced pluripotent stem cells (iPSC) and human embryonic stem cells). For example, the engineered cell is an immune cell. For example, the engineered cell is a primary cell. For example, the engineered cell is a B cell, monocyte, natural killer cell, basophil, eosinophil, neutrophil, dendritic cell, macrophage, T cell, NKT cell, or a combination thereof. The engineered cell may be an autologous cell or an allogeneic cell. For example, the engineered cell is derived from human PBMC cells.

In one embodiment, the immune cell is selected from: B cell, monocyte, natural killer cell, basophil, eosinophil, neutrophil, dendritic cell, macrophage, T cell, NKT cell, stem cell-derived immune effector cell, or a combination thereof.

In one embodiment, the engineered cell is a T cell. In one embodiment, the engineered cell is an allogeneic T cell. In one embodiment, the engineered cell is a stem cell-derived T cell. T cells can be cytotoxic T cells, helper T cells, αβT, or γδT, CD4+/CD8+ double-positive T cells, CD4+ T cells, CD8+ T cells, CD4/CD8 double-negative T cells, CD3+ T cells, naïve T cells, effector T cells, cytotoxic T cells, helper T cells, memory T cells, regulatory T cells, Th0 cells, Th1 cells, Th2 cells, Th3 (Treg) cells, Th9 cells, Th17 cells, Thαβ helper cells, Tfh cells, stem cell-like central memory TSCM cells, central memory TCM cells, effector memory TEM cells, or effector memory TEMRA cells. In one example, the T cell is a cytotoxic T cell. In some embodiments, the genetically engineered T cell provided by the present invention is isolated. In one example, the genetically engineered T cell provided by the present invention is substantially purified.

In one example, the engineered cell provided by the present invention is derived from cells isolated from a subject. As used in the present invention, genetically engineered cells derived from source cells refer to genetically engineered cells obtained by acquiring source cells and performing genetic manipulation on the source cells. The source cells may be from a natural source. For example, the source cells may be primary cells isolated from a subject. The source cells may also be cells that have been passaged or genetically manipulated in vitro.

In one example, the genetically engineered cells provided by the present invention are derived from cells isolated from the human body. Immune effector cells (e.g., T cells) can be obtained from many sources, comprising peripheral blood mononuclear cells, bone marrow, lymph node tissue, umbilical cord blood, thymus tissue, tissue from sites of infection, ascites, pleural effusion, spleen tissue, and tumors. In one example, T cell lines available in the art may be used. In one example, the genetically engineered cells provided by the present invention are derived from cells isolated from peripheral blood. In one example, the genetically engineered cells provided by the present invention are derived from cells isolated from bone marrow. In one example, the genetically engineered cells provided by the present invention are derived from cells isolated from peripheral blood mononuclear cells (PBMC).

In one example, the engineered cells provided by the present invention are derived from cells differentiated *in vitro* from stem cells or progenitor cells. In one example, the stem cells or progenitor cells are selected from the group consisting of T cell progenitors, hematopoietic stem/progenitor cells, hematopoietic multipotent progenitor cells, embryonic stem cells, and induced pluripotent cells. In one example, the genetically engineered cells provided by the present invention are derived from cells differentiated *in vitro* from T cell progenitors. In one example, the genetically engineered cells provided by the present invention are derived from cells differentiated *in vitro* from hematopoietic stem/progenitor cells. In one example, the genetically engineered cells provided by the present invention are derived from cells differentiated *in vitro* from hematopoietic multipotent progenitor cells. In one example, the genetically engineered cells provided by the present invention are derived from cells differentiated *in vitro* from embryonic stem cells. In one example, the genetically engineered cells provided by the present invention are derived from cells differentiated *in vitro* from induced pluripotent cells.

In one example, the engineered cells further comprise: low or no expression of endogenous TCR, B2M, HLA-I, HLA-II, NKG2A, FAS, and/or CD58. In one example, the engineered cells further comprise low or no expression of endogenous TCR and B2M. In one example, the engineered cells further comprise low or no expression of endogenous FAS. In one example, the engineered cells further comprise low or no expression of endogenous CD58. In one example, the engineered cells further comprise low or no expression of endogenous NKG2A. In one example, the engineered cells further comprise no expression of endogenous TCR and B2M. In one example, the engineered cells further comprise no expression of endogenous TCR/B2M/NKG2A. In one example, the engineered cells further comprise no expression of endogenous TCR/B2M/FAS. In one example, the engineered cells further comprise no expression of endogenous TCR/B2M/CD58.

In one example, the present invention provides a method for genetically engineering cells by transferring the polynucleotide provided by the present invention into cells using gene editing. If desired, techniques such as nucleases, transcription activator-like effector nucleases (TALENs), zinc finger nucleases (ZFNs), clustered regularly interspaced short palindromic repeats (CRISPRs), homologous recombination, non-homologous end joining, microhomology-mediated end joining, and homology-mediated end joining can be used for site-specific integration.

In one example, endogenous TCR/B2M, TCR/B2M/NKG2A, TCR/B2M/FAS, or TCR/B2M/CD58 are knocked out by CRISPR/Cas9 technology. In one example, the sgRNA sequences targeting TRAC, B2M, FAS, and NKG2A are shown as SEQ ID NO: 35, 36, 37, and 38, respectively.

For illustration, in some embodiments, the present invention provides engineered T cells expressing chimeric receptors, wherein the engineered T cells exhibit no expression of endogenous TCR/B2M, and optionally no expression of NKG2A, FAS, and/or CD58; the chimeric receptor comprises, from N-terminus to C-terminus, an extracellular domain, a transmembrane domain, and an intracellular signaling domain; wherein the extracellular region comprises an antibody that binds CD19 and CD20 disclosed herein, the transmembrane domain comprises, for example, the transmembrane domain of CD28 or CD8, and the intracellular signaling domain comprises, for example, the intracellular signaling domain of CD137, CD28, or CD3 ζ . The extracellular domain and transmembrane domain may be connected by, for example, a CD8 hinge region, a CD28 hinge region, or an IgG spacer region or a fragment thereof. The engineered cell may further comprise a chimeric receptor that binds immune cell markers.

In one example, the engineered cell expressing the chimeric receptor of this application can bind target cells expressing CD19 and/or target cells expressing CD20. For example, the engineered cell of this application can bind NK cells. In one example, the engineered cell of this application can bind CD19 and/or CD20 positive cells and also bind NK cells. For example, the engineered cell can bind target cells expressing NK cell markers (e.g., CD7, NKG2A, CD38, CD94, CS1, TIGIT, NKG2DL). For example, the engineered cell disclosed herein expressing chimeric receptors targeting CD19 and CD20 also expresses chimeric receptors targeting NKG2A and/or TIGIT. For example, the engineered cell disclosed herein expressing chimeric receptors targeting CD19 and CD20 also expresses chimeric receptors targeting NKG2A. For example, the engineered cell disclosed herein expressing chimeric receptors targeting CD19 and CD20 also expresses chimeric receptors targeting CD38. For example, the engineered cell disclosed herein expressing chimeric receptors targeting CD19 and CD20 also expresses chimeric receptors targeting CD7. For example, the engineered cell disclosed herein expressing chimeric receptors targeting CD19 and CD20 also expresses chimeric receptors targeting NKG2A and FasL. For example, the engineered cell disclosed herein expressing chimeric receptors targeting CD19 and CD20 also expresses chimeric receptors targeting CD38 and FasL.

In one example, the engineered cell expressing the chimeric receptor of this application does not induce host versus graft rejection. In one example, in the presence of host immune cells (e.g., NK cells), the engineered cell expressing the chimeric receptor of this application has longer survival time and/or enhanced expansion capacity. For example, the engineered cell can kill host immune cells. For example, the engineered cell can kill host NK cells. For example, the engineered cell can kill allogeneic NK cells. For example, the engineered cell can resist killing by host NK cells. For example, the engineered cell can resist killing by allogeneic NK cells. For example, compared to cells not expressing the chimeric receptor of this application, the engineered cell expressing the chimeric receptor exhibit significantly enhanced ability to kill pathological cells.

In one example, the engineered cell exhibits prolonged survival time or enhanced expansion capacity in the presence of host immune cells. In one example, the engineered cell has inhibitory or killing effect on host immune cells. In one example, the engineered cell has inhibitory or killing effect on host T cells or NK cells. In one example, the engineered cell has inhibitory or killing effect on host NK cells. In one example, the engineered cell can enhance the survival, proliferation, and the killing effect on pathological cells of another engineered cell introduced into the subject before, simultaneously with, or after them.

In one example, in the presence of host immune cells (e.g., NK cells), the engineered cell expressing binding CD19, CD20, and NK cell markers (e.g., NKG2A, CD38, CD94, CS1, TIGIT, NKG2DL) has longer survival time and/or stronger expansion capacity. For example, the engineered cell has stronger ability to kill host NK cells. For example, the engineered cell has stronger ability to kill allogeneic NK cells. For example, the engineered cell has stronger ability to resist the killing by host NK cells. For example, the engineered cell has stronger ability to resist the killing by allogeneic NK cells.

In one example, the engineered cell expressing the chimeric receptor also expresses immune checkpoint inhibitors. Immune checkpoint inhibitors comprise any agent that blocks, inhibits, or reduces the activity or function of inhibitory pathways of the immune system. Such inhibitors may comprise small molecule inhibitors or antibodies or antigen-binding fragments thereof that bind to and block or inhibit immune checkpoint receptors, ligands, and/or receptor-ligand interactions. Exemplary immune checkpoint molecules targeted for blocking, inhibition, regulation, enhancement, and/or stimulation include but are not limited to: PD-1 (CD279), PD-L1 (CD274, B7-H1), PDL2 (CD273, B7-DC), CTLA-4, LAG-3 (CD223), TIM-3, 4-1BB (CD137), 4-1BBL (CD137L), GITR (TNFRSF18, AITR), CD40, OX40 (CD134, TNFRSF4), CXCR2, tumor-associated antigens (TAA), B7-H3, B7-H4, BTLA, HVEM, GAL9, B7H3, B7H4, VISTA, KIR, 2B4 (a member of the CD2 family and expressed on all NK, γδ, and memory CD8+ (αβ) T cells), CD160 (also known as BY55), CGEN-15049, CEACAM (e.g., CEACAM-1, CEACAM-3, and/or CEACAM-5), TIGIT, LAIR1, CD160, 2B4, CD80, CD86, B7-H3 (CD276), B7-H4 (VTCN1), HVEM (TNFRSF14 or CD270), KIR, A2aR, MHC I class, MHC II class, GAL9, adenosine, and transforming growth factor receptor (TGFR; e.g., TGFRβ). Immune checkpoint inhibitors comprise antibodies or antigen-binding fragments thereof or other binding proteins that bind to and block or inhibit and/or enhance or stimulate the activity of one or more of any of the aforementioned molecules.

### Transduction

The present invention provides a method for transducing viral vectors into cells (e.g., immune effector cells), which involves activation and transduction of the cells to be transduced. Activation and transduction of the cells can be performed simultaneously, i.e., the input composition comprising the cells to be transduced, stimulants for the cells to be transduced, and viral vector particles carrying recombinant nucleic acids encoding the chimeric receptor of the present invention are co-incubated. Alternatively, activation can be performed first, followed by transduction, such as incubating the input composition comprising the cells to be transduced and stimulants for the cells to be transduced for activation, then adding viral vector particles carrying recombinant nucleic acids encoding the chimeric receptor of the present invention for incubation and transduction. The total time for activation and transduction of recombinant nucleic acids is controlled to be completed within 72 hours, preferably within 48 hours, or within 36 hours, or within 24 hours. In some embodiments, the provided method involves incubating and/or contacting retroviral vector particles (e.g., lentiviral vectors) with a population of cells (e.g., immune cells, such as T cells), wherein the T cells are activated and/or stimulated using ex vivo cell activation reagents (e.g., anti-CD3/anti-CD28 reagents) before and/or during and/or after contacting or incubating the cells with viral particles. Preferably, cells are activated first, then viral transduction is performed. In one example, when the input composition comprising the cells to be transduced, stimulants for the cells to be transduced, and viral vector particles carrying recombinant nucleic acids encoding the chimeric receptor of the present invention are co-incubated, the incubation time does not exceed 72 hours before harvesting to obtain the output composition, which comprises cells transduced with recombinant nucleic acids; preferably, the incubation time can be 1 hour to 72 hours; more preferably, the incubation time is 2 hours to 48 hours; even more preferably, the incubation time is 2 hours to 36 hours; even more preferably, the incubation time is 12 hours to 36 hours; even more preferably, the incubation time is 12 hours to 24 hours; even more preferably, the incubation time is 15 hours to 24 hours. In one example, after purification treatments such as washing and centrifugation, the output composition can be used to prepare pharmaceutical formulations without further *in vitro* expansion and culture, i.e., *in vitro* expansion is not necessary for the medicinal products prepared from the output composition before being used in a subject (or patient).

The following specific embodiments further illustrate the present invention. It should be understood that these embodiments are only for illustrating the present invention and not for limiting its scope. Experimental methods not specifically described in the following embodiments are generally carried out under conventional conditions as described in J. Sambrook et al., Molecular Cloning: A Laboratory Manual, Third Edition, Science Press, 2002, or according to the conditions recommended by the manufacturer.

The biological materials used in the examples of this application are shown in Table 5.

| Name | Source |
|---|---|
| T cells | Isolated from healthy donor primary PBMC |
| NK cells | Isolated from healthy donor primary PBMC |
| Nalm6 | ATCC |
| Daudi | Cell Bank, Chinese Academy of Sciences, Shanghai |
| NPG mice | Beijing Vital River Laboratory Animal Technology Co., Ltd., genotype NOD.Cg-Prkdcscid I12rgtm1 Vst/Vst |

### Example 1. Vector Construction

Vectors expressing the chimeric receptors disclosed in this application were constructed using conventional molecular biology techniques. Full-length DNA was synthesized, to construct the bispecific CAR expression vectors CAR6-12 as shown in Table 3, which were integrated into the pRRLSIN vector (Addgene), respectively. Lentiviruses corresponding to the above expression vectors were prepared using conventional molecular biology techniques.

Full-length DNA was synthesized to construct the expression vectors CAR19.1, 19.2, 19.3 binding CD19, and/or CAR20.4, 20.5, 20.6, 20.7, 20.8 binding CD20 as shown in Tables 1 and 2, which were integrated into the pRRLSIN vector (Addgene), respectively. Lentiviruses corresponding to the above expression vectors were prepared using conventional molecular biology techniques.

### Example 2. Construction of CAR-T Cells

CAR-T cells were prepared using conventional methods in the field: PBMC cells were collected from donors, activated with anti-CD3 and anti-CD28 antibody-coated magnetic beads (Life Technologies, 40203D), and then infected with lentivirus comprising the above vectors to obtain CAR-T1-12 cells expressing the corresponding chimeric receptors. T cells not transduced with virus were considered as UTD.

For each CAR-T sample, 1×10⁵ cells were collected and incubated with 5µg/ml biotinylated anti-FMC63 antibody (Acrobiosystems, FM3-BY54) and anti-Fab antibody (Jackson, 109-066-006) at room temperature for 1 hour, then Streptavidin BV421 (Biolegend, 405225, 1:200 dilution) antibody was added and incubated at room temperature for 30 minutes. CAR positivity was detected by FACS.

### Example 3. Detection of CD19 and CD20 Expression in Human B Lymphoblastic Leukemia Cell Nalm6 and Human Burkitt's Lymphoma Cell Daudi

Nalm6 cells (Nalm6-Luci) and Daudi cells (Daudi-Luci) expressing exogenous luciferase were constructed using conventional molecular biology techniques. CD19 and CD20 expression in different cells were detected by flow cytometry (FACS) using anti-CD19 APC-H7 antibody (BD, 560727) and anti-CD20 APC antibody (Invitrogen, 17-0209-4). Figure 2 shows that Daudi expresses CD19 and CD20. Nalm6 expresses CD19.

### Example 4. In Vitro Cytotoxicity Activity of Dual-Target CAR-T Cells Against Lymphoma Cells

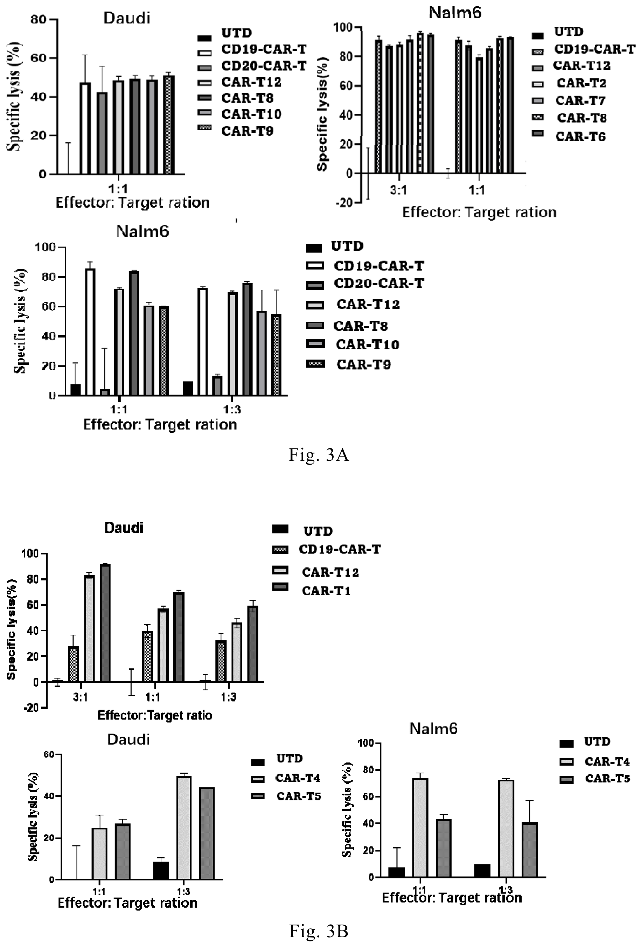

Daudi and Nalm6 cells were seeded in 96-well plates at 10,000 cells/well. CAR-T cells were co-incubated with Daudi and Nalm6 cells at effector-to-target ratios of 3:1, 1:1, or 1:3 for 18 hours, and the cytotoxicity of CAR-T cells against lymphoma cells was detected by FACS. Figures 3A and 3B show that CD19/CD20 dual-target CAR-T1, 2, 4, 5, 6, 7, 8, 9, and 10 cells all significantly killed tumor cells Daudi and Nalm6.

### Example 5. Cytokine Secretion by Dual-Target CAR-T Cells After Co-incubation with Tumor Cells

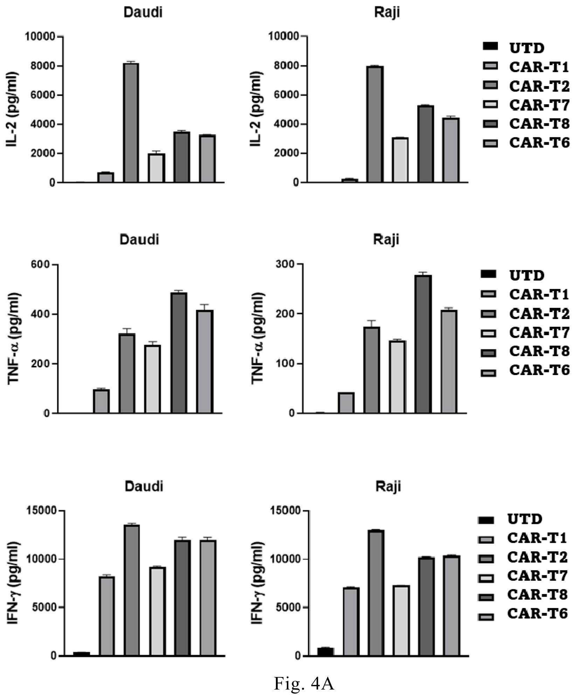

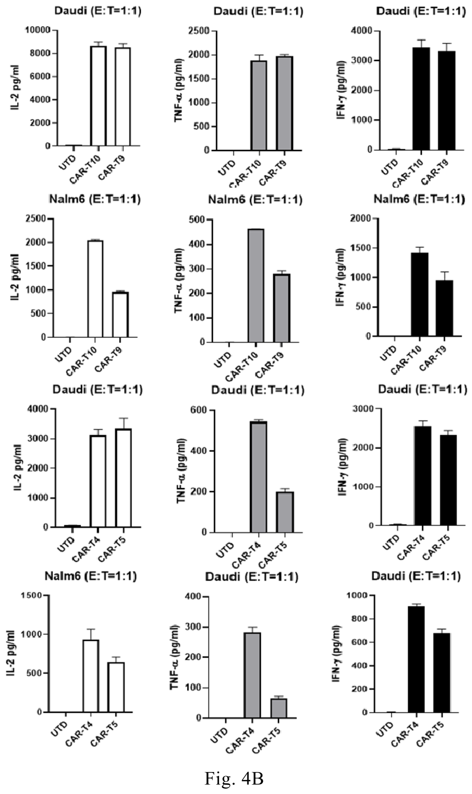

Daudi and Nalm6 lymphoma cells were seeded in 96-well plates at 40,000 cells/well. CD19/CD20 dual-target CAR-T cells were co-incubated with Daudi and Nalm6 cells at an effector-to-target ratio of 1:1 for 18 hours, and secretion levels of interferon-γ (IFN-γ), IL-2, and TNF-α in the supernatant were detected using the CBA method (BD, 558264). Figures 4A and 4B show that CD19/CD20 dual-target CAR-T1, 2, 4, 5, 6, 7, 8, 9, and 10 cells all secreted high levels of IFN-γ, IL-2, and TNF-α.

### Example 6. Dual-Target CAR-T cells Therapy for Subcutaneous Transplanted Lymphoma in NPG Mice

On D0 (day 0), each NPG mouse was subcutaneously injected with 3×10⁶ Daudi cells, 5 mice per group. On D12, the average tumor volume was about 130 mm³. On D13, each mouse was injected via the tail vein with 3×10⁶ CD19/CD20 dual-target CAR-T cells.

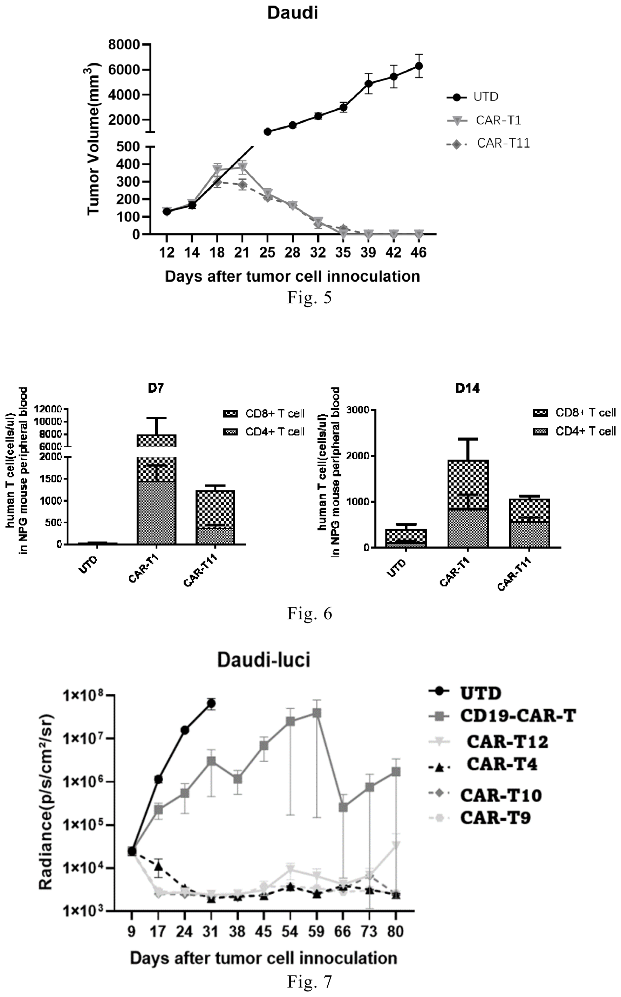

Figure 5 shows that on D34, compared to the UTD group, the tumor volume inhibition rate in the CAR-T1 and CAR-T11 groups was 100%; all 5 mice in the CAR-T1 group had completely tumor regression, and 2 mice in the CAR-T11 group had tumor regression.

### Example 7. Detection of Human T Cell Survival in Mouse Peripheral Blood

After CAR-T treatment as described in Example 6, 4 mice in each group were randomly selected, and peripheral blood was collected from each group. 50µL from each blood sample was taken for detection using anti-CD45 APC-H7 (1ul/test, BD, 560178), CD3 percp-cy5.5/CD4FITC/CD8PE (10ul/test, BD, 340298) antibodies, and BD absolute counting tubes (BD, Cat:340334, Lot:21258, Beads Number:49450/tube). Figure 6 shows that on Day 7 and 14 after CAR-T cell infusion, surviving human T cells were detected in the peripheral blood of mice in the CAR-T1 and CAR-T11 groups.

### Example 8. Treatment of Orthotopic Lymphoma in NPG Mice with Dual-Target CAR-T cells

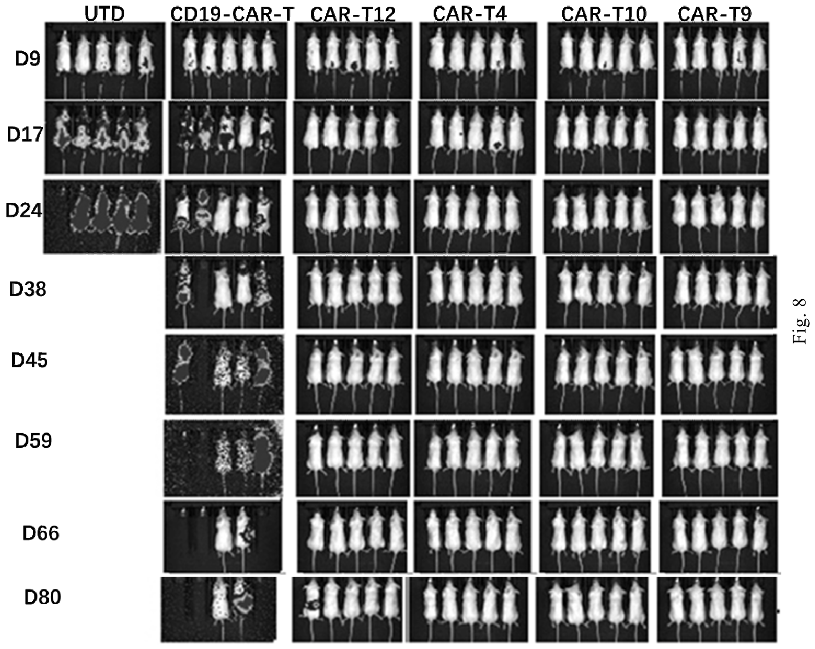

On D0, each NPG mouse was injected via the tail vein with 3×10⁶ Daudi-Luci cells, 5 mice per group. Tumor photon intensity was observed at different time points using fluorescence imaging (IVIS LUMINAII SYSTEM). On D9, when the photon intensity reached an average of 2.47×10⁴ p/s/cm²/sr, each mouse was injected via the tail vein with 1×10⁶ CAR-T cells. Figures 7 and 8 show that by D80, in the CD19-CAR-T group, 3 mice died and 1 mouse showed sustained tumor fluorescence rebound starting from D31; in the CAR-T12 group, 1 mouse showed sustained tumor fluorescence rebound starting from D45; while the CAR-T4, CAR-T9, and CAR-T10 groups showed the best tumor suppression effect: no tumor rebound occurred, all mice survived and were in good condition.

### Example 9. Preparation of UCAR-T Cells

The sgRNA sequences targeting TRAC, B2M, FAS, and NKG2A, respectively (as shown in SEQ ID NO: 35, 36, 37, 38, respectively) (CARsgen Diagnostics) were synthesized *in vitro,* which were used to knock out endogenous TCR/B2M or TCR/B2M/NKG2A in CAR-T9 and CAR-T12 cells constructed in Example 2 by CRISPR/Cas9 technology (Cas9 protein, (Kactus Biosystems (Shanghai) Co., Ltd., CAS-EE109). CAR-T9 (DKO), CAR-T9 (TKO), and CAR-T12 (DKO) cells were obtained sequentially by magnetic bead sorting (Miltenyi, 130-048-801). UTD cells with TCR/B2M/NKG2A being knockout but not transduced were used as controls. For each CAR-T sample, 1×10⁵ cells were collected and incubated with 5µg/ml biotinylated anti-FMC63 antibody (Acrobiosystems, FM3-BY54) or anti-Fab antibody (Jackson, 109-066-006) or NKG2A protein (CARsgen Diagnostics, KD1140) at room temperature for 1 hour, then Streptavidin BV421 (Biolegend, 405225, 1:200 dilution) or Streptavidin PE antibody (Biolegend, 405225) was added and incubated at room temperature for 30 minutes. CAR positivity was detected by FACS.

### Example 10. Treatment of Orthotopic Lymphoma in NPG Mice with Dual-Target CAR-T cells

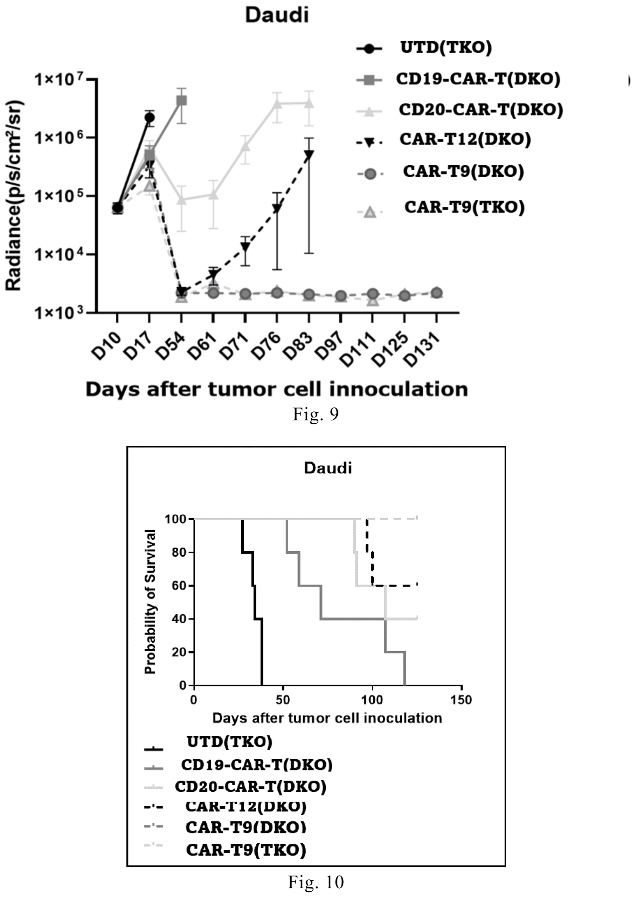

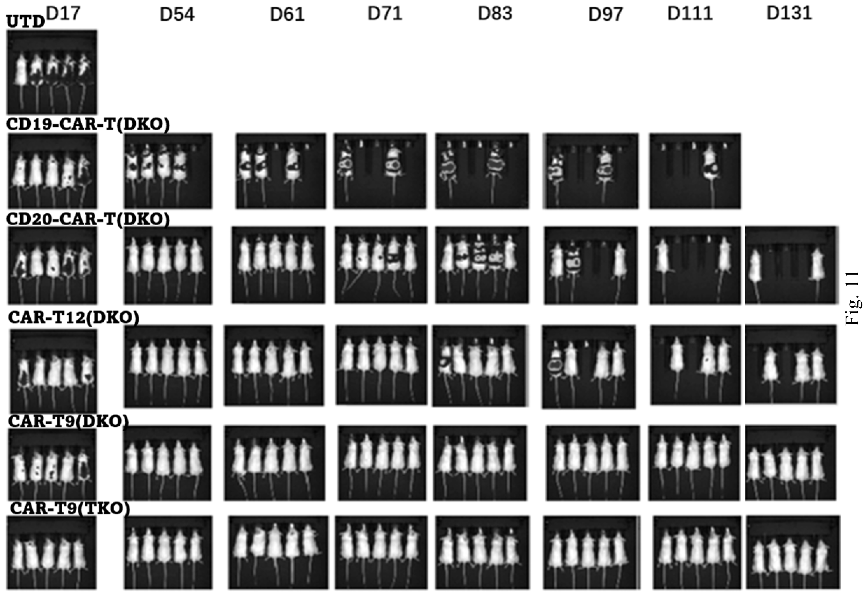

On D0, each NPG mouse was injected via the tail vein with 3×10⁶ Daudi-Luci cells, 5 mice per group. Tumor photon intensity was observed at different time points using fluorescence imaging (IVIS LUMINAII SYSTEM). On D10 (photon intensity reached an average of 6.4×10⁴p/s/cm²/sr), each mouse was injected via the tail vein with 1×10⁶ UCAR-T cells. Figures 9, 10, and 11 show that by D131, all mice in the CD19-CAR-T (DKO) group died; 3 mice in the CD20-CAR-T (DKO) group died; 2 mice in the CAR-T12 (DKO) group died. The CAR-T9 (DKO) and CAR-T9 (TKO) groups showed the best tumor suppression effect: no tumor rebound occurred, all mice survived and were in good condition.

### Example 11. Construction of Vectors and Cells Targeting Immune Cells

Vectors expressing chimeric receptors targeting immune cell markers were constructed using conventional molecular biology techniques. Full-length DNA was synthesized to construct the expression vectors NKG2A-28z and NKG2A-s28z as shown in Table 4, which were integrated into the pRRLSIN vector (Addgene).

CAR9/NKG2A(1)T cells and CAR9/NKG2A(2)T cells were constructed as described in Example 2.

### Example 12. Preparation of CD19/CD20-NKG2A UCAR-T Cells

TCR/B2M/NKG2A in CAR9/NKG2A(1)T cells or CAR9/NKG2A(2)T cells was knocked out as described in Example 9 to construct CAR9/NKG2A(1)-tko cells and CAR9/NKG2A(2)-tko cells. UTD cells with TCR/B2M/NKG2A being knockout but not transduced were used as controls. For each CAR-T sample, 1×10⁵ cells were collected and incubated with 5µg/ml biotinylated anti-FMC63 antibody (Acrobiosystems, FM3-BY54) or anti-Fab antibody (Jackson, 109-066-006) or NKG2A protein (Kexing Diagnostics, HD1140) at room temperature for 1 hour, then Streptavidin BV421 (Biolegend, 405225, 1:200 dilution) or Streptavidin PE antibody (eBioscience, 12-4317-87) was added and incubated at room temperature for 30 minutes. CAR positivity was detected by FACS.

### Example 13. In Vitro Cytotoxicity Activity of CD19/CD20-NKG2A UCAR-T Cells Against Lymphoma Cells

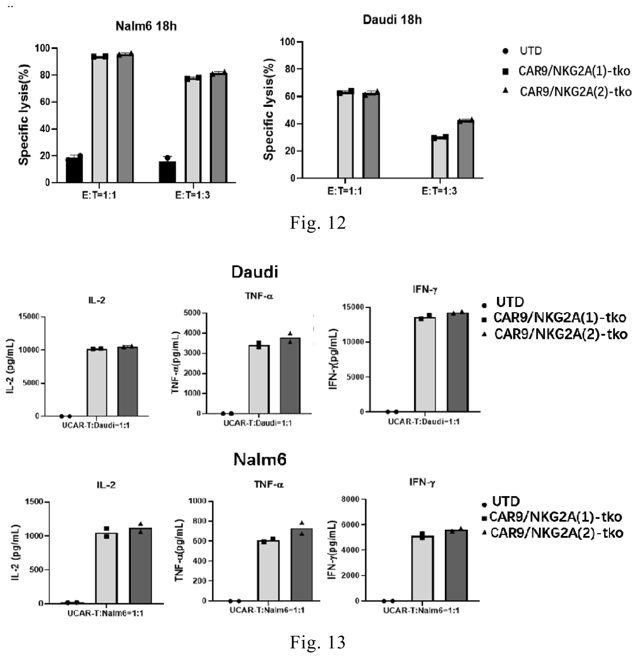

30,000 Daudi and Nalm6 cells were seeded in each well of a 96-well plate, respectively. CAR9/NKG2A(1)-tko or CAR9/NKG2A(2)-tko cells were co-incubated at an effector-to-target ratio of 1:1 or 1:3 for 18 hours, then 7-AAD (BD, 559925) and Counting Beads (Invitrogen, C36995) were added for the quantification of both cell types. The remaining cell numbers of both cell types were counted by flow cytometry, and the cytotoxicity was calculated as: % cytotoxicity = (1 - experimental group/control group) *100. Figure 12 shows that both CAR9/NKG2A(1)-tko and CAR9/NKG2A(2)-tko cells significantly killed lymphoma cells.

### Example 14. Cytokine Secretion by CD19/CD20-NKG2A UCAR-T Cells After Co-incubation with Tumor Cells

CD19/CD20-NKG2A UCAR-T cells were co-cultured with Daudi or Nalm6 for 18 hours, and the supernatant was collected. The secretion of IL-2, TNF-α, and IFN-γ cytokines were detected using the CBA-Human Soluble Protein Master Buffer Kit (Cat: 558265). Figure 13 shows that after co-incubation with tumor cells, both CAR9/NKG2A(1)-tko and CAR9/NKG2A(2)-tko cells secreted high levels of IL-2, TNF-α, and IFN-γ cytokines.

### Example 15. In vitro killing on human primary NK cells by CD19/CD20-NKG2A UCAR-T cells

PBMC cells were collected from donors, and NK cells were isolated using CD56 magnetic beads (Miltenyi, 130-050-401) according to the manufacturer's instructions.

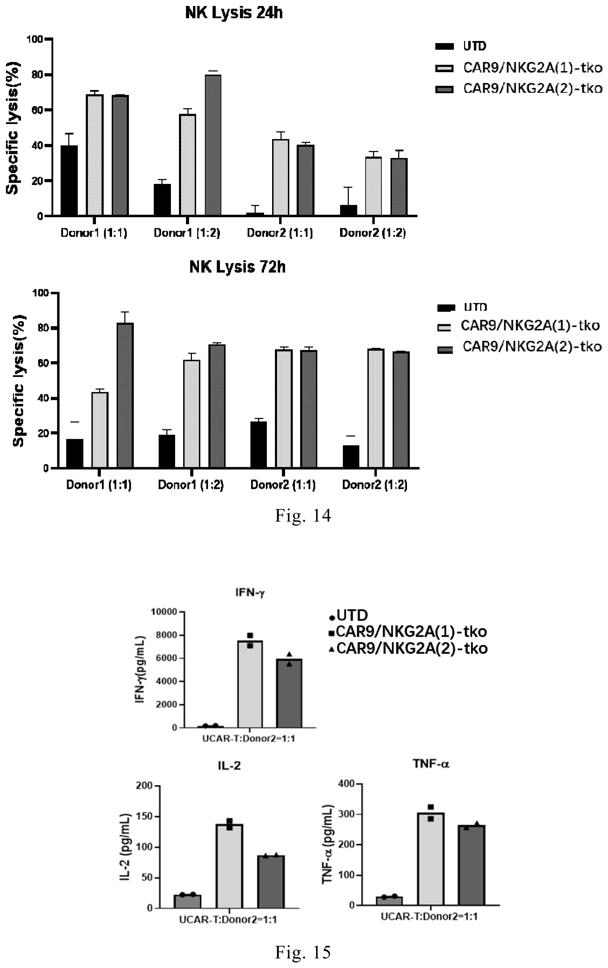

3 × 10⁴ CD19/CD20-NKG2A UCAR-T cells were collected and co-cultured with the above human primary NK cells at a ratio of 1:1 or 1:2 for 24 h or 72 h. B2M-KO T cells were labeled with HLA-ABC (Thermo Fisher, 17-9983-42), NK cells were labeled with CD56 (BD 555516); 7-AAD fluorescent dye (BD, 559925) was used to distinguish dead and live cells. Counting Beads (Invitrogen, C36995) were added, and the number of NK cells were detected by flow cytometry to calculate the lysis rate of NK cells. Figure 14 shows that after co-incubation with NK cells for 24 or 72 hours, both CAR9/NKG2A(1)-tko and CAR9/NKG2A(2)-tko cells can significantly kill NK cells.

### Example 16. Cytokines IL-2, TNF-α, and IFN-γ Secretion by CD19/CD20-NKG2A UCAR-T cells after co-incubation with NK cells

The supernatant was collected after co-culturing CD19/CD20-NKG2AUCAR-T cells with human primary NK cells for 24 hours, and the secretion of IL-2, TNF-α, and IFN-γ cytokines was detected using the CBA-Human Soluble Protein Master Buffer Kit (Cat: 558265). Figure 15 shows that after co-incubation with NK cells, both CAR9/NKG2A(1)-tko and CAR9/NKG2A(2)-tko cells can secrete high levels of IL-2, TNF-α, and IFN-γ cytokines.

### Example 17. Rapid preparation process for CAR-T cells targeting CD19 and CD20

T cells were activated with magnetic beads (Miltenyi, 170-076-156) for 1, 2, 3, 4, 5, 6, 7, 8, 9, 10, 11, 12, 13, 14, 15, 16, 17, 18, 19, 20, 21, 22, 23, or 24 hours, then transduced with virus comprising CAR targeting CD19 and CD20 (prepared by conventional molecular biology techniques) for a transduction time of 1, 2, 3, 4, 5, 6, 7, 8, 9, 10, 11, 12, 13, 14, 15, 16, 17, 18, 19, 20, 21, 22, 23, or 24 hours. The transduced T cells were collected, cryopreserved, or seeded in 24-well plates at a cell density of about 1 × 10⁶ cells/ml and cultured for more than 24 hours before cryopreservation.

The embodiments of the present invention comprise using any single embodiment or combining any embodiment or part thereof with any other embodiment. Furthermore, it should be understood that after reading the above teachings of the present invention, those skilled in the art may make various changes or modifications to the invention; these equivalents also fall within the scope defined by the appended claims of this application.

### Sequence information

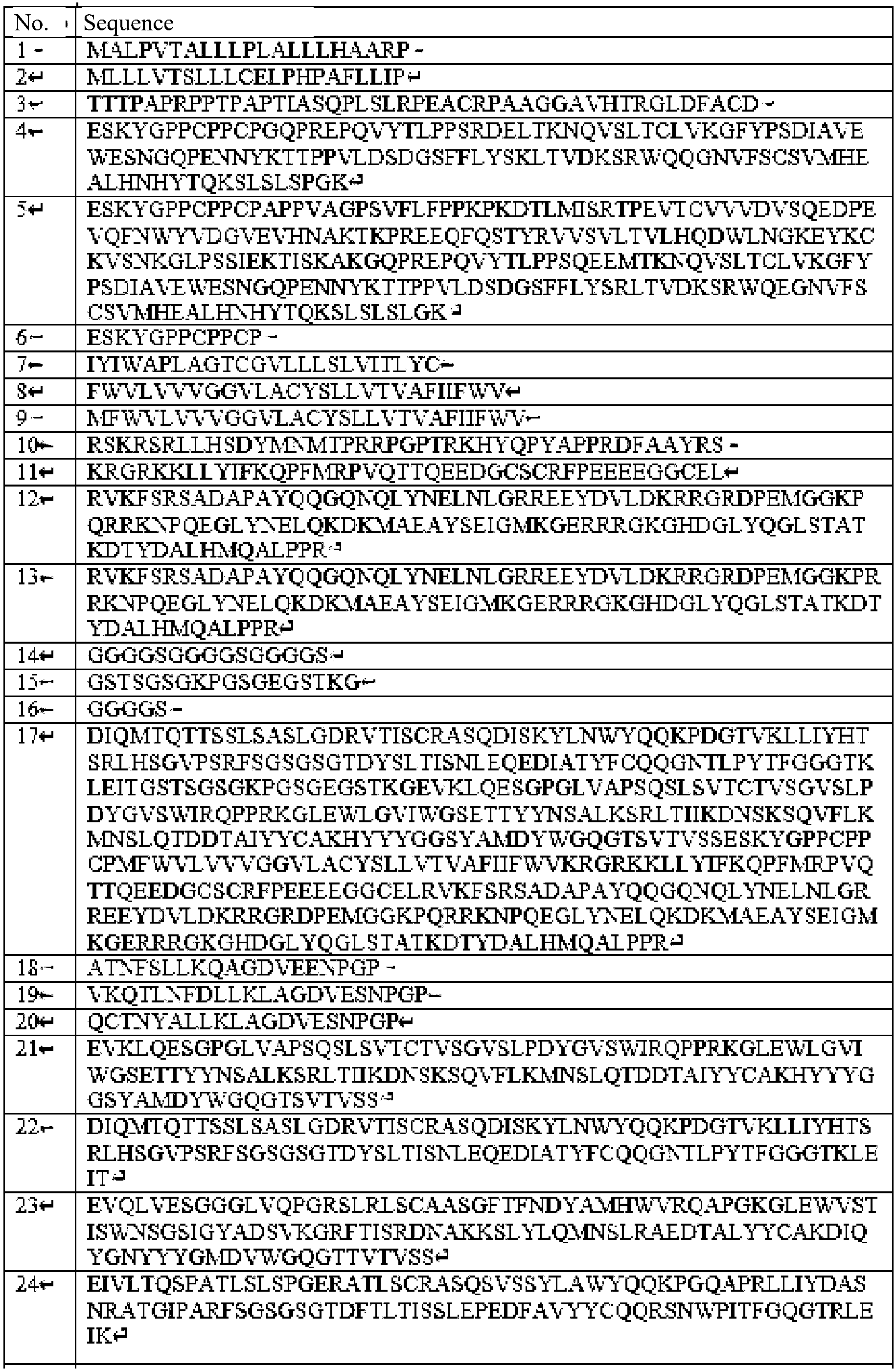

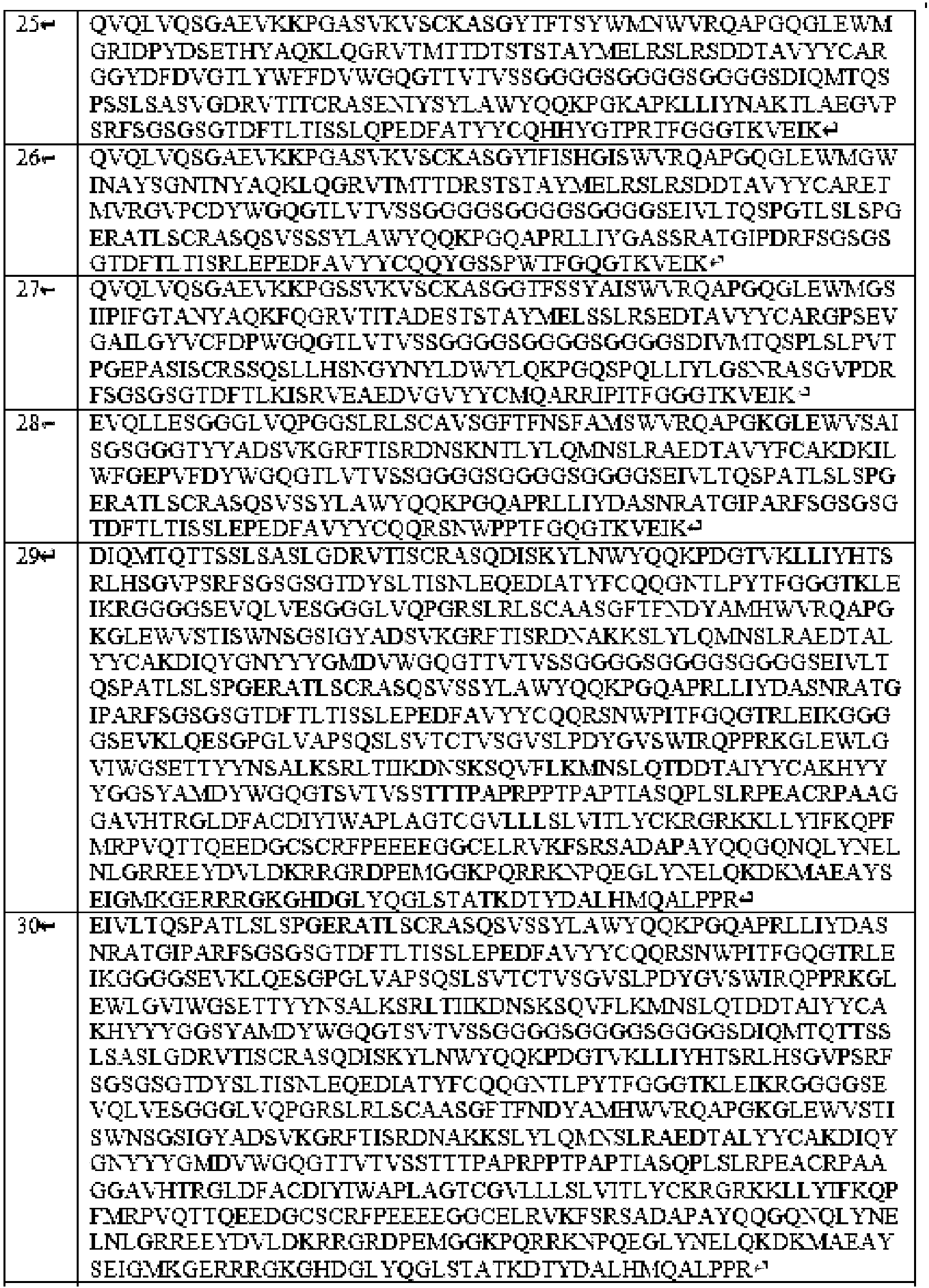

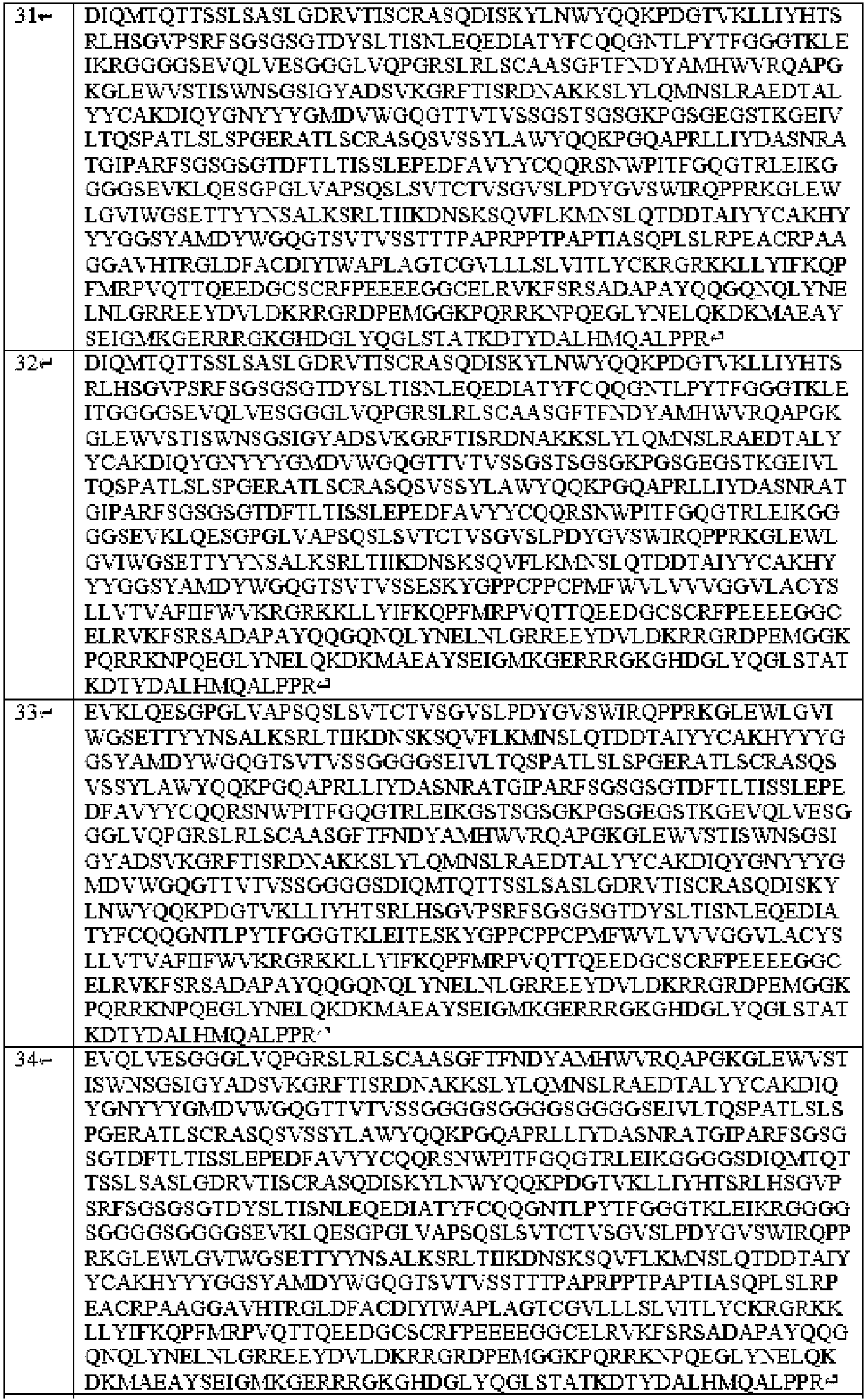

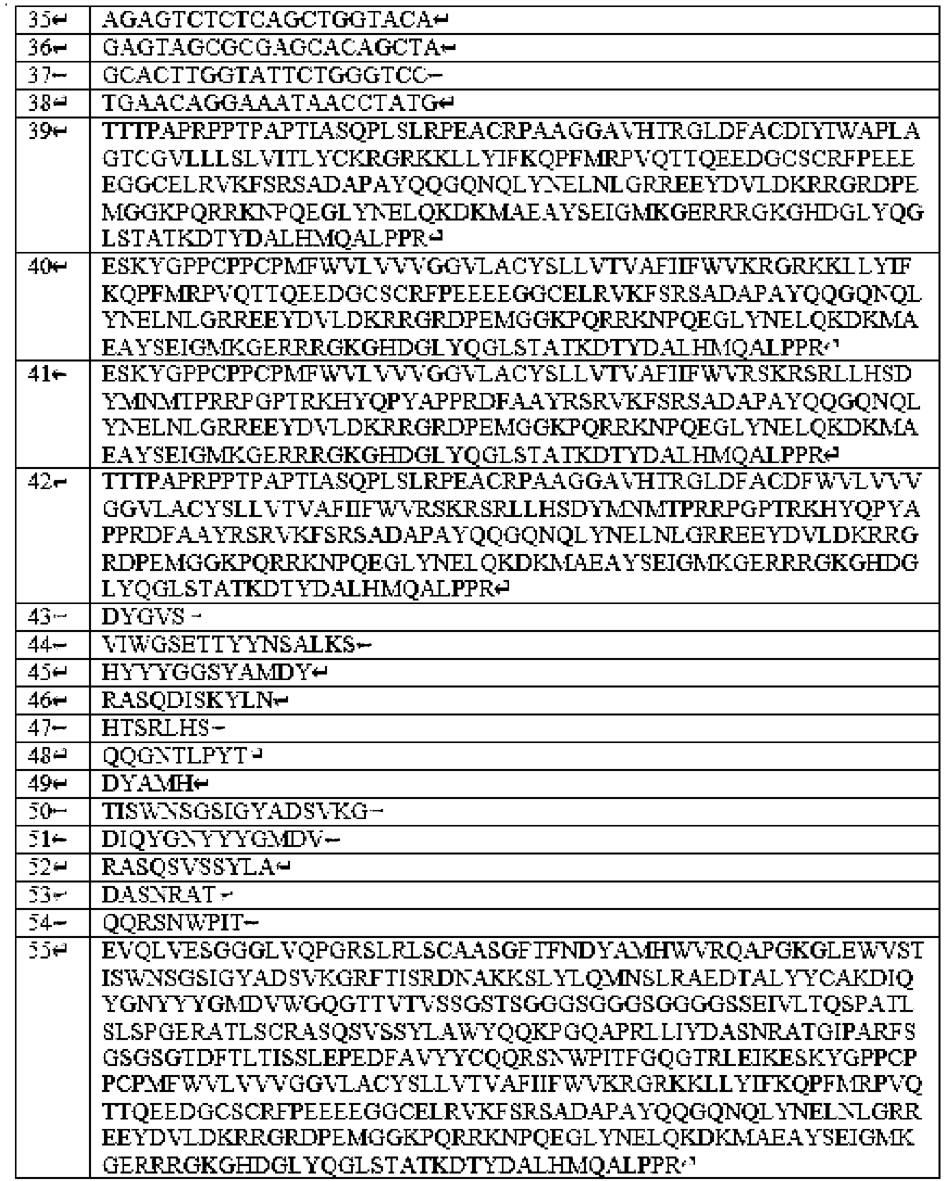

All references mentioned in this invention are incorporated herein by reference, as if each reference were individually cited as such. Furthermore, it should be understood that after reading the above teachings of the present invention, those skilled in the art may make various changes or modifications to the invention; these equivalents also fall within the scope defined by the appended claims of this application.

## Claims

1. A bispecific chimeric receptor, wherein the chimeric receptor comprises an antigen-binding domain, a transmembrane domain, and an intracellular domain; the antigen-binding domain comprises an anti-CD19 antibody heavy chain variable region (V_{H1}) and light chain variable region (V_{L1}), an anti-CD20 antibody heavy chain variable region (V_{H2}) and light chain variable region (V_{L2}), and V_{H1}, V_{L1}, V_{H2}, V_{L2} are connected in a loop structure.

2. The chimeric receptor according to claim 1, wherein the antigen-binding domain comprises:
(1) the anti-CD20 antibody heavy chain variable region (V_{H2}) and light chain variable region (V_{L2}) connected as antibody 2, and the anti-CD19 antibody heavy chain variable region (V_{H1}) and light chain variable region (V_{L1}) are respectively connected to both ends of antibody 2; or
(2) the anti-CD19 antibody heavy chain variable region (V_{H1}) and light chain variable region (V_{L1}) connected as antibody 1, and the anti-CD20 antibody heavy chain variable region (V_{H2}) and light chain variable region (V_{L2}) are respectively connected to both ends of antibody 1;
preferably, from the N-terminus to the C-terminus, the antibody 2 comprises V_{H2}, V_{L2} or V_{L2}, V_{H2}; or from the N-terminus to the C-terminus, the antibody 1 comprises V_{H1}, V_{L1} or V_{L1}, V_{H1}.

3. The chimeric receptor according to claim 2, wherein, from the N-terminus to the C-terminus, the antigen-binding domain of the chimeric receptor is represented by any one of the following formulas: (1) V_{L1}-V_{H2}-I-V_{L2}-V_{H1}, (2) V_{H1}-V_{H2}-I-V_{L2}-V_{L1}, (3) V_{H1}-V_{L2}-I-V_{H2}-V_{L1}, (4) V_{L1}-V_{L2}-I-V_{H2}-V_{H1}, (5) V_{L2}-V_{H1}-I-V_{L1}-V_{H2}, (6) V_{H2}-V_{H1}-I-V_{L1}-V_{L2}, (7) V_{H2}-V_{L1}-I-V_{H1}-V_{L2}, (8) V_{L2}-V_{L1}-I-V_{H1}-V_{H2}, where each "-" independently represents a linker peptide or peptide bond; and I is a flexible linker.

4. The chimeric receptor according to any one of claims 1-3, wherein V_{H1} comprises CDR1, CDR2, and CDR3 having an amino acid sequences that is about 80% to about 100% identity to the amino acid sequences shown in SEQ ID NO:43, SEQ ID NO:44, SEQ ID NO:45, respectively; V_{L1} comprises CDR1, CDR2, and CDR3 having an amino acid sequences that is about 80% to about 100% identity to the amino acid sequences shown in SEQ ID NO:46, SEQ ID NO:47, SEQ ID NO:48, respectively; and/or V_{H2} comprises CDR1, CDR2, and CDR3 having an amino acid sequences that is about 80% to about 100% identity to the amino acid sequences shown in SEQ ID NO:49, SEQ ID NO:50, SEQ ID NO:51, respectively; and V_{L2} comprises CDR1, CDR2, and CDR3 having an amino acid sequences that is about 80% to about 100% identity to the amino acid sequences shown in SEQ ID NO:52, SEQ ID NO:53, SEQ ID NO:54, respectively;
preferably, V_{H1} and V_{L1} have amino acid sequences that is about 80% to about 100% identity to the amino acid sequences shown in SEQ ID NO:21 and SEQ ID NO:22, respectively; and/or V_{H2} and V_{L2} have amino acid sequences that is about 80% to about 100% identity to the amino acid sequences shown in SEQ ID NO:23 and SEQ ID NO:24, respectively.

5. The chimeric receptor according to claim 3, wherein the flexible peptide linker is selected from: a newlinker or (G4S)n, wherein n is 1, 2, 3, 4, or 5; preferably, the flexible peptide linker comprises the amino acid sequences shown in SEQ ID NO:14, SEQ ID NO:15, or SEQ ID NO:16.

6. The chimeric receptor according to any one of claims 1-5, wherein the intracellular domain comprises a costimulatory signaling molecule selected from: the intracellular domain of CD27, CD28, CD137, OX40, CD30, CD40, PD-1, ICOS, or combinations thereof; preferably, the costimulatory signaling molecule is selected from: CD28 (SEQ ID NO:10), or CD137 (SEQ ID NO:11); preferably, the intracellular domain comprises the cytoplasmic signaling domain of CD3ζ; and preferably, the cytoplasmic signaling domain of CD3ζ comprises the amino acid sequences shown in SEQ ID NO:12 or 13.

7. The chimeric receptor according to any one of claims 1-6, wherein the antigen-binding domain is connected to the transmembrane domain via a hinge region, the hinge region is selected from: an IgG1, IgG2, IgG4 hinge region or fragments thereof, a CD8 hinge region or fragments thereof, a CD28 hinge region or fragments thereof; and preferably, the hinge region comprises the amino acid sequence shown in SEQ ID NO:3, 4, 5, or 6.

8. The chimeric receptor according to any one of claims 1-7, wherein the transmembrane domain is selected from: the transmembrane domain of CD28, CD3, CD45, CD4, CD5, CD8, CD9, CD16, CD22, CD33, CD37, CD64, CD80, CD86, CD134, CD137, or CD154; preferably, the transmembrane domain is selected from: CD8, or CD28; and more preferably, the transmembrane domain comprises the amino acid sequence shown in SEQ ID NO:7, 8, or 9.

9. The chimeric receptor according to any one of claims 1-8, wherein the chimeric receptor comprises an amino acid sequence that is about 80% to 100% identity to any one of the amino acid sequences shown in SEQ ID NO:29, 31, 32, 33, or 34.

10. An engineered cell, wherein the engineered cell comprises the chimeric receptor according to any one of claims 1-9.

11. An engineered cell comprising chimeric receptors binding CD19 and CD20, wherein the engineered cell comprises a first chimeric receptor binding CD19 and a second chimeric receptor binding CD20, the structures of the first chimeric receptor binding CD19 and the second chimeric receptor binding CD20 are represented by any one of the following formulas:
(1) L-V_{L1}-V_{H1}-H-TM-C-CD3ζ and L-V_{H2}-V_{L2}-H-TM-C1-CD3ζ
(2) L-V_{L1}-V_{H1}-H-TM-C-CD3ζ and L-V_{L2}-V_{H2}-H-TM-C1-CD3ζ
(3) L-V_{H2}-V_{L2}-H-TM-C-CD3ζ and L-V_{H1}-V_{L1}-H-TM-C1
(4) L-V_{H1}-V_{L1}-H-TM-C-CD3ζ and L-V_{H2}-V_{L2}-H-TM-C1
(5) L-V_{H1}-V_{L1}-H-TM-C-CD3ζ and L-V_{H2}-V_{L2}-H-TM-C1-C2
where each "-" independently represents a linker peptide or peptide bond; L is an optional signal peptide sequence; H is an optional hinge region; TM is a transmembrane domain; C1 is costimulatory signaling molecule 1, C2 is costimulatory signaling molecule 2; CD3ζ is a cytoplasmic signaling sequence derived from CD3ζ; V_{H1} is the anti-CD19 antibody heavy chain variable region; V_{L1} is the anti-CD19 antibody light chain variable region; V_{L2} is the anti-CD20 antibody light chain variable region; V_{H2} is the anti-CD20 antibody heavy chain variable region; "-" is a linker peptide or peptide bond.

12. The engineered cell according to claim 11, wherein V_{H1} comprises CDR1, CDR2, and CDR3 having amino acid sequences that are about 80% to about 100% identity to the amino acid sequences shown in SEQ ID NO:43, SEQ ID NO:44, SEQ ID NO:45, respectively; V_{L1} comprises CDR1, CDR2, and CDR3 having amino acid sequences that are about 80% to about 100% identity to the amino acid sequences shown in SEQ ID NO:46, SEQ ID NO:47, SEQ ID NO:48, respectively; and/or V_{H2} comprises CDR1, CDR2, and CDR3 having amino acid sequences that are about 80% to about 100% identity to the amino acid sequences shown in SEQ ID NO:49, SEQ ID NO:50, SEQ ID NO:51, respectively; V_{L2} comprises CDR1, CDR2, and CDR3 having amino acid sequences that are about 80% to about 100% identity to the amino acid sequences shown in SEQ ID NO:52, SEQ ID NO:53, SEQ ID NO:54, respectively;
preferably, V_{H1} and V_{L1} have amino acid sequences with about 80% to about 100% identity to the amino acid sequences shown in SEQ ID NO:21 and SEQ ID NO:22, respectively; and/or V_{H2} and V_{L2} have amino acid sequences with about 80% to about 100% identity to the amino acid sequences shown in SEQ ID NO:23 and SEQ ID NO:24, respectively.

13. The engineered cell according to claim 11 or 12, wherein the intracellular domain comprises a costimulatory signaling molecule selected from: the intracellular domain of CD27, CD28, CD137, OX40, CD30, CD40, PD-1, ICOS, or combinations thereof; and preferably, the costimulatory signaling molecule is selected from: CD28 (SEQ ID NO:10) and/or CD137 (SEQ ID NO:11).

14. The engineered cell according to any one of claims 11-13, wherein the antigen-binding domain is connected to the transmembrane domain via a hinge region, the hinge region is selected from: an IgG1, IgG2, IgG4 hinge region or fragments thereof, a CD8 hinge region or fragments thereof, a CD28 hinge region or fragments thereof; and preferably, the hinge region comprises the amino acid sequence shown in SEQ ID NO:3, 4, 5, or 6.

15. The engineered cell according to any one of claims 11-14, wherein the transmembrane domain is selected from: the transmembrane domain of CD28, CD3, CD45, CD4, CD5, CD8, CD9, CD16, CD22, CD33, CD37, CD64, CD80, CD86, CD134, CD137, or CD154; and preferably, the transmembrane domain is selected from: CD8 (SEQ ID NO:7), or CD28 (SEQ ID NO:8, 9).

16. The engineered cell according to any one of claims 11-15, wherein the first chimeric receptor and the second chimeric receptor comprise amino acid sequences with about 80% to 100% identity to the amino acid sequences shown in SEQ ID NO:17, 55, respectively.

17. The engineered cell according to any one of claims 10-16, wherein the engineered cell further comprises a third chimeric receptor, the third chimeric receptor binds at least one or two or more immune cell markers; preferably, the immune cell marker is selected from: T cell markers and/or NK cell markers; preferably, the immune cell marker is an NK inhibitory receptor (NKIR); and preferably, the immune cell marker is selected from: CD2, CD3, CD4, CD5, CD7, CD8, CD16a, CD16b, CD25, CD27, CD28, CD30, CD38, CD45, CD48, CD50, CD52, CD56, CD57, CD62L, CD69, CD94, CD100, CD102, CD122, CD127, CD132, CD137, CD138, CD160, CD161, CD178, CD218, CD226, CD244, CD159a (NKG2A), CD159c (NKG2C), NKG2E, CD279, CD314 (NKG2D), CD305, CD335 (NKP46), CD337, CD319 (CS1), TCRα, TCRβ, TIGIT, TRAIL, SLAMF7, NKG2F, NKG2H, NKp30, NKp44, NKp46, NKp80, SLAM family members, L-selectin, natural cytotoxicity receptors NCR1, NCR2, NCR3, or combinations thereof.

18. The engineered cell according to any one of claims 10-17, wherein the third chimeric receptor comprises the amino acid sequences shown in SEQ ID NO:25, 26, 27, 28 and/or 39, 40, 41, 42.

19. The engineered cell according to any one of claims 10-18, wherein the engineered cell further comprises: low expression or no expression of endogenous TCR, B2M, HLA-I, HLA-II, NKG2A, FAS and/or CD58; and preferably, the engineered cell further comprises: no expression of endogenous TCR and B2M, or no expression of endogenous TCR/B2M/NKG2A.

20. The engineered cell according to any one of claims 10-19, wherein the engineered cell is an immune cell, neuron, epithelial cell, endothelial cell, stem cell, or a combination thereof; preferably, the engineered cell is an immune cell selected from: T cell, NK cell, cytotoxic T cell, NKT cell, dendritic cell, macrophage, CIK cell, stem cell-derived immune cell or a combination thereof; and preferably, the engineered cell is an autologous or allogeneic cell.

21. A polynucleotide, wherein the polynucleotide encodes: the chimeric receptor according to any one of claims 1-9, the chimeric receptor according to any one of claims 1-9 and the third chimeric receptor according to any one of claims 17-20, the first and second chimeric receptors according to any one of claims 11-20, the first and second chimeric receptors according to any one of claims 11-20 and the third chimeric receptor according to any one of claims 17-20.

22. A vector, wherein the vector comprises the polynucleotide according to claim 21; and preferably, the chimeric receptor and the third chimeric receptor are located on the same expression vector; or the first and second chimeric receptors are located on the same expression vector.

23. A method for the rapid preparation of engineered cells, wherein the engineered cell comprises the polynucleotide according to claim 21, comprising the following steps: (i) incubating an input composition comprising cells to be transduced and a stimulator for the cells to be transduced for an incubation time of no more than 24 hours, (ii) then adding viral vector particles comprising the polynucleotide according to claim 21 for an incubation time of no more than 24 hours, to obtain the engineered cell; preferably, the vector is derived from a retroviral vector; preferably, the vector is a lentiviral vector; and preferably, no *in vitro* expansion is necessary before the administration to a subject in need thereof.

24. A formulation, wherein the formulation comprises the polynucleotide according to claim 21, the vector according to claim 22, or the chimeric receptor according to any one of claims 1-9, or the engineered cells according to any one of claims 10-20, and a pharmaceutically acceptable carrier, diluent, or excipient.

25. A medicament for preventing or treating diseases, wherein the medicament comprises the polynucleotide according to claim 21, the vector according to claim 22, or the chimeric receptor according to any one of claims 1-9, or the engineered cells according to any one of claims 10-20.

26. The chimeric receptor according to any one of claims 1-9, or the engineered cell according to any one of claims 10-20, for use in killing or inhibiting CD19 and/or CD20 positive pathological cells; preferably, for use in preparing a medicament for treating autoimmune diseases or inflammatory diseases; preferably, for use in treating, preventing, or ameliorating autoimmune diseases or inflammatory diseases in a subject in need thereof; preferably, the autoimmune diseases are selected from: multiple sclerosis, autoimmune liver disease, type 1 diabetes, systemic lupus erythematosus (SLE), rheumatoid arthritis (RA), ankylosing spondylitis (AS), Sjögren's syndrome (SS), polymyositis/dermatomyositis; preferably, for use in preparing a medicament for treating tumors; preferably, for use in treating, preventing, or ameliorating tumors in a subject in need thereof; and preferably, the tumors comprise: leukemia, lymphoma, myeloma.

27. A multifunctional immune effector cell, wherein the immune effector cell comprises a chimeric receptor binding CD19 and CD20, or comprises a first chimeric receptor binding CD19 and a second chimeric receptor binding CD20, the structure of the chimeric receptor binding CD19 and CD20 is represented by any one of the following formulas:
(1) L-V_{L1}V_{H2}-I-V_{L2}-V_{H1}-H-TM-C-CD3ζ
(2) L-V_{L2}-V_{H1}-I-V_{L1}-V_{H2}-H-TM-C-CD3ζ
(3) L-V_{H1}-V_{L2}-I-V_{H2}-V_{L1}-H-TM-C-CD3ζ
(4) L-V_{H2}-V_{L2}-I-V_{L1}-V_{H1}-H-TM-C-CD3ζ;
the structures of the first chimeric receptor binding CD19 and the second chimeric receptor binding CD20 are represented by any one of the following formulas:
(5) L-V_{L1}-V_{H1}-H-TM-C-CD3ζ and L-V_{H2}-V_{L2}-H-TM-C-CD3ζ
(6) L-V_{L1}-V_{H1}-H-TM-C-CD3ζ and L-V_{L2}-V_{H2}-H-TM-C-CD3ζ
(7) L-V_{H2}-V_{L2}-H-TM-C-CD3ζ and L-V_{H1}-V_{L1}-H-TM-C
(8) L-V_{H1}-V_{L1}-H-TM-C-CD3ζ and L-V_{H2}-V_{L2}-H-TM-C
(9) L-V_{H1}-V_{L1}-H-TM-C-CD3ζ and L-V_{H2}-V_{L2}-H-TM-C-CD3ζ;
where each "-" independently represents a linker peptide or peptide bond; L is an optional signal peptide sequence; I is a flexible linker; H is an optional hinge region; TM is a transmembrane domain; C is a costimulatory signaling molecule; CD3ζ is a cytoplasmic signaling sequence derived from CD3ζ; V_{H1} is the CD19 antibody heavy chain variable region; V_{L1} is the CD19 antibody light chain variable region; V_{L2} is the CD20 antibody light chain variable region; V_{H2} is the CD20 antibody heavy chain variable region; "-" is a linker peptide or peptide bond.

28. A polynucleotide, wherein the polynucleotide encodes a chimeric receptor binding both CD19 and CD20, or encodes a first chimeric receptor binding CD19 and a second chimeric receptor binding CD20; wherein the polynucleotide encoding the chimeric receptor binding both CD19 and CD20 is represented by any one of the following formulas:
(1) L-V_{L1}V_{H2}-I-V_{L2}-V_{H1}-H-TM-C-CD3ζ
(2) L-V_{L2}-V_{H1}-I-V_{L1}-V_{H2}-H-TM-C-CD3ζ
(3) I,-V_{H1}-V_{L2}-I-V_{H2}-V_{L1}-H-TM-C-CD3ζ
(4) L-V_{H2}-V_{L2}-I-V_{L1}-V_{H1}-H-TM-C-CD3ζ;
wherein the polynucleotide encoding the first chimeric receptor binding CD19 and the second chimeric receptor binding CD20 is represented by any one of the following formulas:
(5) L-V_{L1}-V_{H1}-H-TM-C-CD3ζ-2A-L-V_{H2}-V_{L2}-H-TM-C-CD3ζ
(6) L-V_{L1}-V_{H1}-H-TM-C-CD3ζ-2A-L-V_{L2}-V_{H2}-H-TM-C-CD3ζ
(7) L-V_{H2}-V_{L2}-H-TM-C-CD3ζ-2A-L-V_{H1}-V_{L1}-H-TM-C
(8) L-V_{H1}-V_{L1}-H-TM-C-CD3ζ-2A-L-V_{H2}-V_{L2}-H-TM-C
(9) L-V_{H1}-V_{L1}-H-TM-C-CD3ζ-2A-L-V_{H2}-V_{L2}-H-TM-C-CD3ζ;
wherein each "-" independently represents a linker peptide or peptide bond; L is an optional nucleic acid component encoding a signal peptide; I is a nucleic acid component encoding a flexible linker; H is an optional nucleic acid component encoding hinge region; TM is a nucleic acid component encoding a transmembrane domain; C is a nucleic acid component encoding a costimulatory signaling molecule; CD3ζ is a cytoplasmic signaling sequence derived from CD3ζ; 2A is an optional nucleic acid component encoding 2A self-cleaving peptide; V_{H1} is the CD19 antibody heavy chain variable region; V_{L1} is the CD19 antibody light chain variable region; V_{L2} is the CD20 antibody light chain variable region; V_{H2} is the CD20 antibody heavy chain variable region; "-" is a linker peptide or peptide bond.
